(19) Europäisches Patentamt / European Patent Office / Office européen des brevets

(11) **EP 4 053 511 A1**

(12) **EUROPEAN PATENT APPLICATION**
published in accordance with Art. 153(4) EPC

(43) Date of publication:
**07.09.2022 Bulletin 2022/36**

(21) Application number: **20881000.2**

(22) Date of filing: **29.10.2020**

(51) International Patent Classification (IPC):
**G01F 1/66** (2022.01)   **A61B 5/026** (2006.01)
**A61B 5/0285** (2006.01)

(52) Cooperative Patent Classification (CPC):
**A61B 5/026; A61B 5/0285; G01F 1/66**

(86) International application number:
**PCT/JP2020/040593**

(87) International publication number:
**WO 2021/085525 (06.05.2021 Gazette 2021/18)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **31.10.2019 JP 2019198577**

(71) Applicant: **Kyocera Corporation
Kyoto-shi Kyoto 612-8501 (JP)**

(72) Inventors:
- **TODA Keisuke
  Kyoto-shi, Kyoto 612-8501 (JP)**
- **MATSUNAGA Shougo
  Kyoto-shi, Kyoto 612-8501 (JP)**

(74) Representative: **Viering, Jentschura & Partner
mbB
Patent- und Rechtsanwälte
Am Brauhaus 8
01099 Dresden (DE)**

(54) **MEASURING DEVICE, MEASURING SYSTEM, MEASURING METHOD AND PROGRAM**

(57)   A measurement device includes a light emitter, a light receiver, an extractor, and a processor. The light emitter illuminates an illumination target having an internal space through which a fluid flows. The light receiver receives coherent light including light scattered by the illumination target and outputs a signal corresponding to intensity of the coherent light. The extractor extracts a direct-current component from the signal output from the light receiver at a temporal change in strength of the signal. The processor calculates a calculation value for a flow state of the fluid by performing a process on the signal output from the light receiver. The process includes correction using a value of signal strength of the direct-current component and calculation of a frequency spectrum for the signal at the temporal change in the signal strength.

FIG. 7A

FIG. 7B

EP 4 053 511 A1

## Description

### FIELD

The present disclosure relates to a measurement device, a measurement system, a measurement method, and a program.

### BACKGROUND

Known devices for quantitatively measuring the flowing states of fluids include measurement devices that measure the flow rate and the flow velocity of a fluid with an optical method using, for example, a laser blood flowmeter (refer to, for example, Japanese Patent No. 5806390).

### BRIEF SUMMARY

One or more aspects of the disclosure are directed to a measurement device, a measurement system, a measurement method, and a program.

A measurement device according to an aspect includes a light emitter, a light receiver, an extractor, and a processor. The light emitter illuminates an illumination target having an internal space through which a fluid flows. The light receiver receives coherent light including light scattered by the illumination target and outputs a signal corresponding to intensity of the coherent light. The extractor extracts a direct-current component from the signal output from the light receiver at a temporal change in strength of the signal. The processor calculates a calculation value for a flow state of the fluid by performing a process on the signal output from the light receiver. The process includes correction using a value of signal strength of the direct-current component and calculation of a frequency spectrum for the signal at the temporal change in the signal strength.

A measurement device according to another aspect includes a light emitter, a light receiver, an extractor, and a processor. The light emitter illuminates an illumination target having an internal space through which a fluid flows. The light receiver receives coherent light including light scattered by the illumination target and outputs a signal corresponding to intensity of the coherent light. The extractor extracts a direct-current component from the signal output from the light receiver at a temporal change in strength of the signal. The processor calculates a frequency spectrum for the signal output from the light receiver at the temporal change in the signal strength and calculates a quantitative value for a flow state of the fluid with a computation using a value of signal strength based on the frequency spectrum and a value of signal strength of the direct-current component.

A measurement system according to an aspect includes a light emitter, a light receiver, an extractor, and a processor. The light emitter illuminates an illumination target having an internal space through which a fluid flows. The light receiver receives coherent light including light scattered by the illumination target and outputs a signal corresponding to intensity of the coherent light. The extractor extracts a direct-current component from the signal output from the light receiver at a temporal change in strength of the signal. The processor calculates a calculation value for a flow state of the fluid by performing a process on the signal output from the light receiver. The process includes correction using a value of signal strength of the direct-current component and calculation of a frequency spectrum for the signal at the temporal change in the signal strength.

A measurement system according to another aspect includes a light emitter, a light receiver, an extractor, and a processor. The light emitter illuminates an illumination target having an internal space through which a fluid flows. The light receiver receives coherent light including light scattered by the illumination target and outputs a signal corresponding to intensity of the coherent light. The extractor extracts a direct-current component from the signal output from the light receiver at a temporal change in strength of the signal. The processor calculates a frequency spectrum for the signal output from the light receiver at the temporal change in the signal strength and calculates a quantitative value for a flow state of the fluid with a computation using a value of signal strength based on the frequency spectrum and a value of signal strength of the direct-current component.

A measurement method according to an aspect includes illuminating, extracting, and calculating. The illuminating includes illuminating, with a light emitter, an illumination target having an internal space through which a fluid flows, receiving, with a light receiver, coherent light including light scattered by the illumination target, and outputting, with the light receiver, a signal corresponding to intensity of the coherent light. The extracting includes extracting, with an extractor, a direct-current component from the signal output from the light receiver at a temporal change in strength of the signal. The calculating includes calculating, with a processor, a calculation value for a flow state of the fluid by performing a process on the signal output from the light receiver. The process includes correction using a value of signal strength of the direct-current component extracted by the extractor and calculation of a frequency spectrum for the signal at the temporal change in the signal strength.

A measurement method according to another aspect includes illuminating, extracting, and calculating. The illuminating includes illuminating, with a light emitter, an illumination target having an internal space through which a fluid flows, receiving, with a light receiver, coherent light including light scattered by the illumination target, and outputting, with the light receiver, a signal corresponding to intensity of the coherent light. The extracting includes extracting, with an extractor, a direct-current component from the signal output from the light receiver at a temporal change in strength of the signal. The calculating includes calculating, with a processor, a frequency spectrum for the signal output from the light receiver at the temporal change in the signal strength, and calculating, with the processor, a quantitative value for a flow state of the fluid with a computation using a value of signal strength based on the frequency spectrum and a value of signal strength of the direct-current component extracted by the extractor.

A program according to an aspect is a program executable by a processor included in a measurement device to cause the measurement device to function as the measurement device according to any one of the above aspects.

**BRIEF DESCRIPTION OF DRAWINGS**

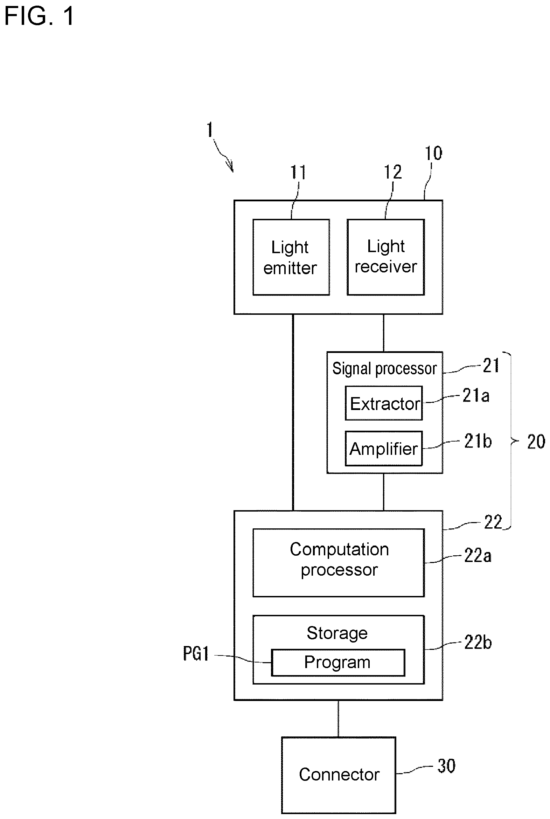

FIG. 1 is a schematic block diagram of a measurement device according to a first embodiment.

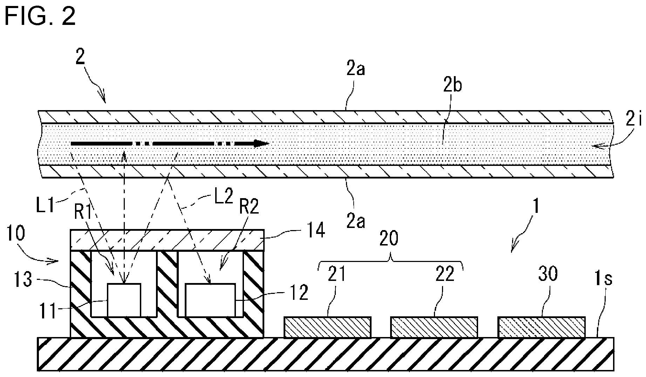

FIG. 2 is a schematic partial cross-sectional view of the measurement device according to the first embodiment.

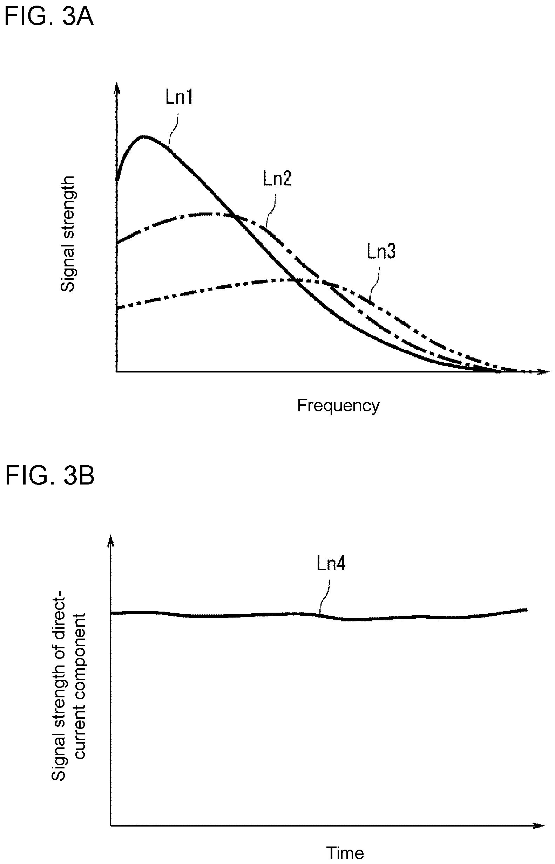

FIG. 3A is a graph for illumination light with first intensity showing a curve Ln1 indicating an example frequency spectrum of coherent light from an illumination target through which a fluid having a flow quantitative value of a relatively small value Vq1 flows, a curve Ln2 indicating an example frequency spectrum of coherent light from an illumination target through which a fluid having a flow quantitative value of a relatively intermediate value Vq2 flows, and a curve Ln3 indicating an example frequency spectrum of coherent light from an illumination target through which a fluid having a flow quantitative value of a relatively large value Vq3 flows, and FIG. 3B is a graph for illumination light with first intensity showing an example direct-current component in the signal strength of coherent light from an illumination target through which a fluid flows.

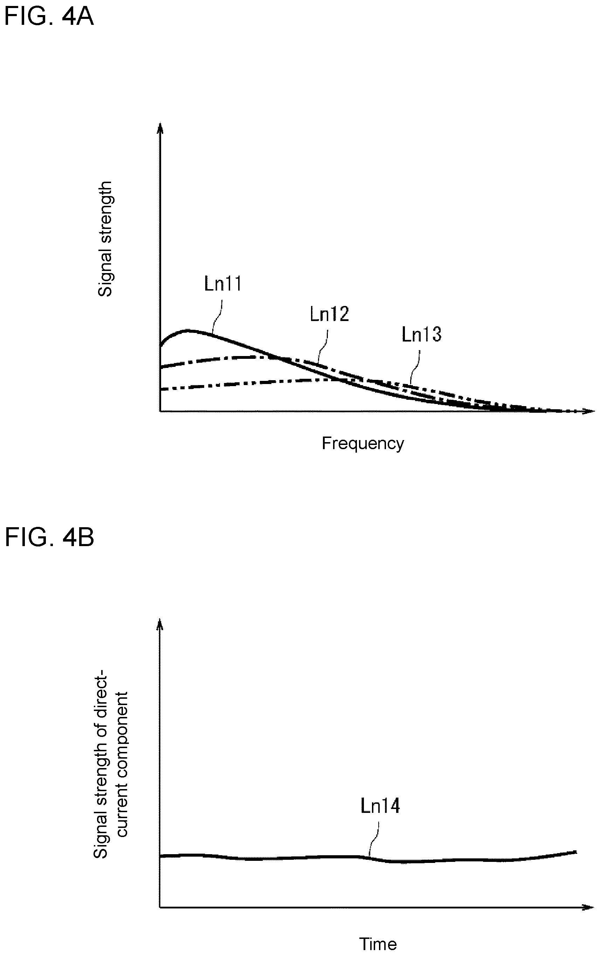

FIG. 4A is a graph for illumination light with second intensity lower than the first intensity showing a curve Ln11 indicating an example frequency spectrum of coherent light from an illumination target through which a fluid having a flow quantitative value of a relatively small value Vq1 flows, a curve Ln12 indicating an example frequency spectrum of coherent light from an illumination target through which a fluid having a flow quantitative value of a relatively intermediate value Vq2 flows, and a curve Ln13 indicating an example frequency spectrum of coherent light from an illumination target through which a fluid having a flow quantitative value of a relatively large value Vq3 flows, and FIG. 4B is a graph for illumination light with second intensity lower than the first intensity showing an example direct-current component in the signal strength of coherent light from an illumination target through which a fluid flows.

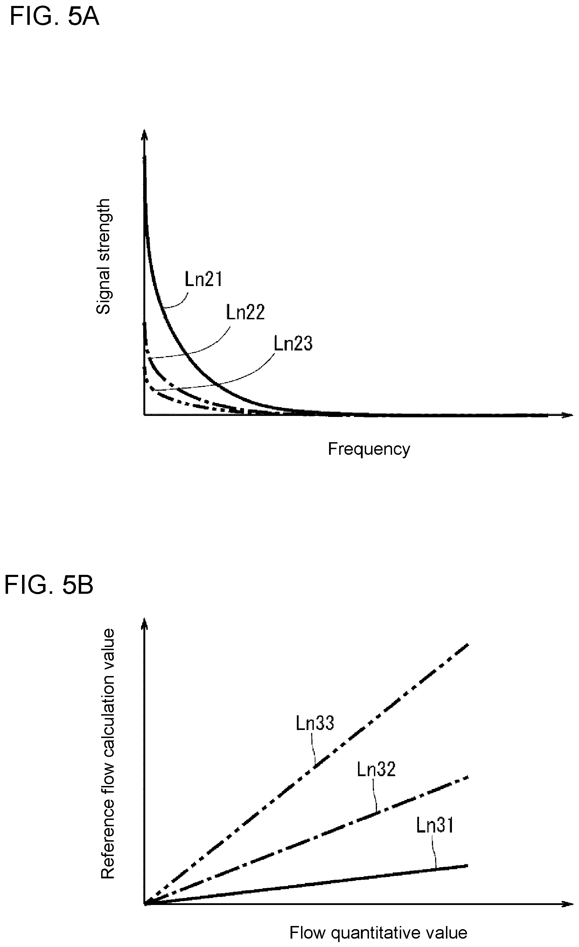

FIG. 5A is a graph for a flow quantitative value with a predetermined value showing a curve Ln21 indicating an example frequency spectrum calculated for illumination light with first intensity, a curve Ln22 indicating an example frequency spectrum calculated for illumination light with second intensity lower than the first intensity, and a curve Ln23 indicating an example frequency spectrum calculated for illumination light with third intensity lower than the second intensity, and FIG. 5B is a graph showing a line Ln31 indicating an example relationship between a flow quantitative value and a reference flow calculation value for illumination light with first intensity, a line Ln32 indicating an example relationship between a flow quantitative value and a reference flow calculation value for illumination light with second intensity lower than the first intensity, and a line Ln33 indicating an example relationship between a flow quantitative value and a reference flow calculation value for illumination light with third intensity lower than the second intensity.

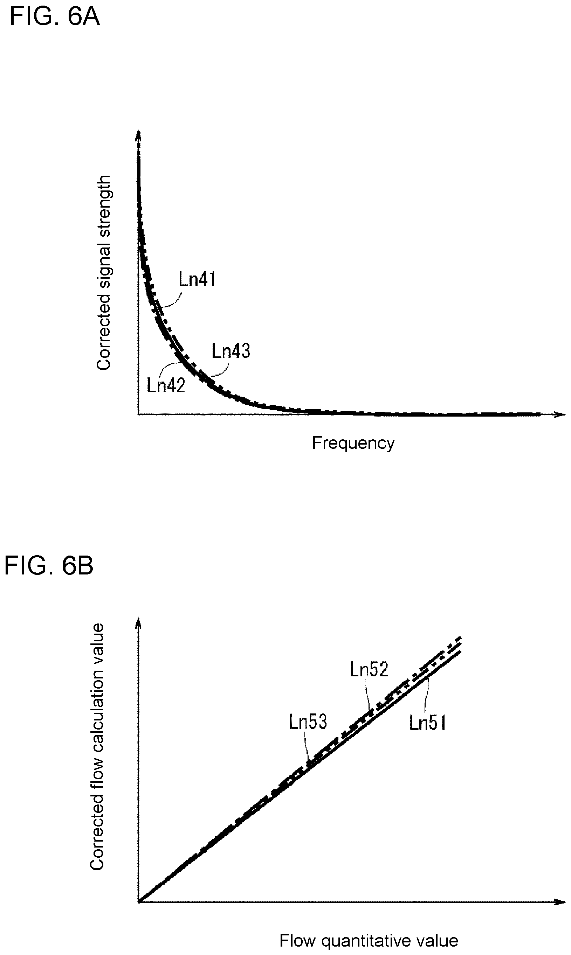

FIG. 6A is a graph for a flow quantitative value with a predetermined value showing a curve Ln41 as an example corrected frequency spectrum calculated for illumination light with first intensity, a curve Ln42 as an example corrected frequency spectrum calculated for illumination light with second intensity lower than the first intensity, and a curve Ln43 as an example corrected frequency spectrum calculated for illumination light with third intensity lower than the second intensity, and FIG. 6B is a graph showing a line Ln51 indicating an example relationship between a flow quantitative value and a corrected flow calculation value for illumination light with first intensity, a line Ln52 indicating an example relationship between a flow quantitative value and a corrected flow calculation value for illumination light with second intensity lower than the first intensity, and a line Ln53 indicating an example relationship between a flow quantitative value and a corrected flow calculation value for illumination light with third intensity lower than the second intensity.

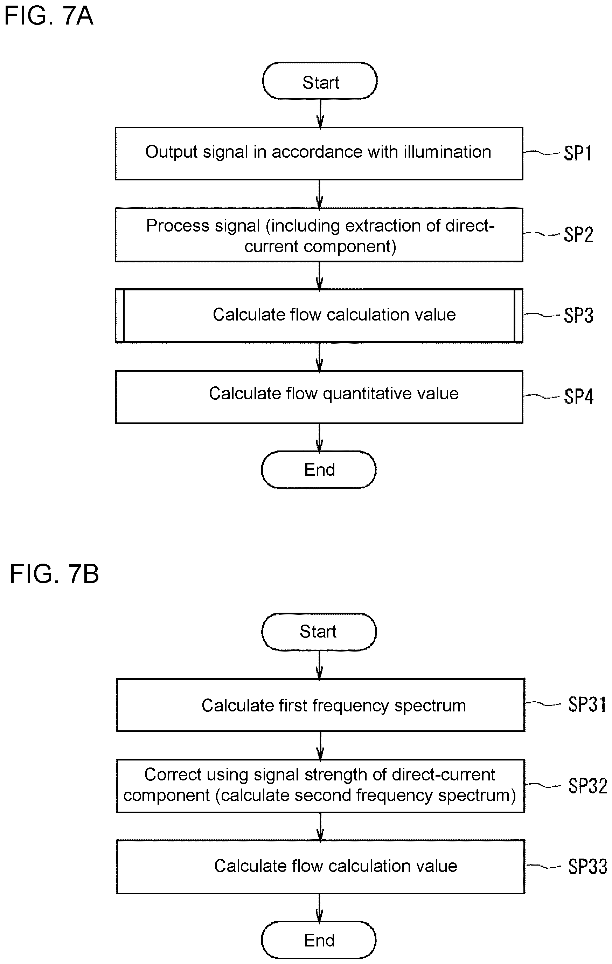

FIG. 7A is a flowchart showing an example operation of a measurement device according to the first embodiment, and FIG. 7B is a flowchart showing a first example operation of the measurement device according to the first embodiment calculating a flow calculation value.

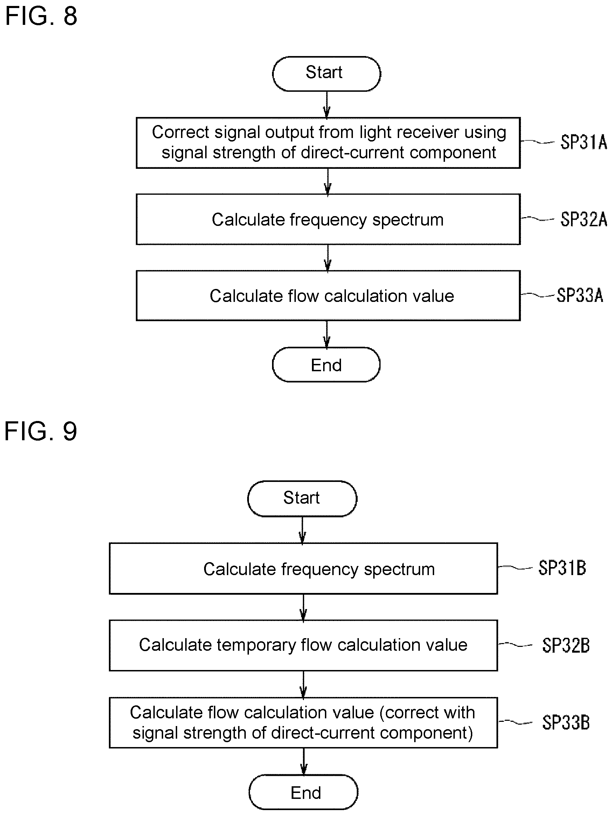

FIG. 8 is a flowchart showing a second example operation of the measurement device according to the first embodiment calculating a flow calculation value.

FIG. 9 is a flowchart showing a third example operation of the measurement device according to the first embodiment calculating a flow calculation value.

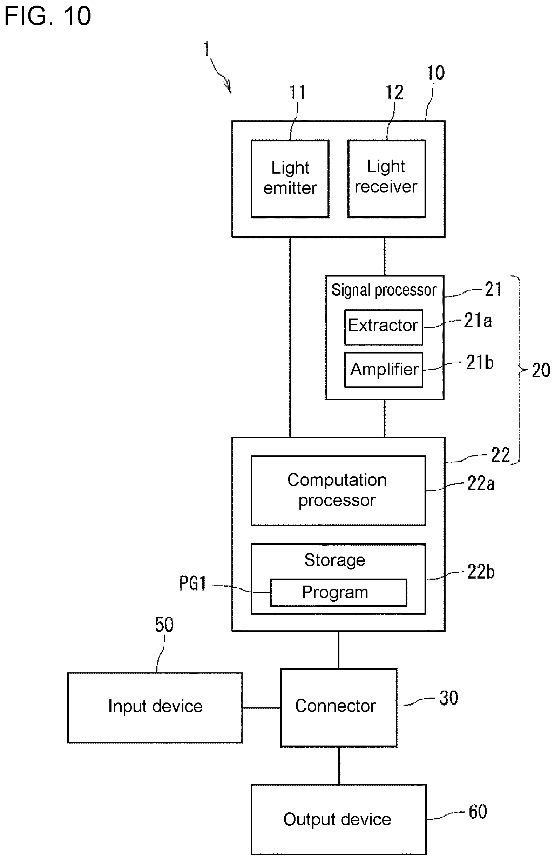

FIG. 10 is a schematic block diagram of a measurement device according to a second embodiment.

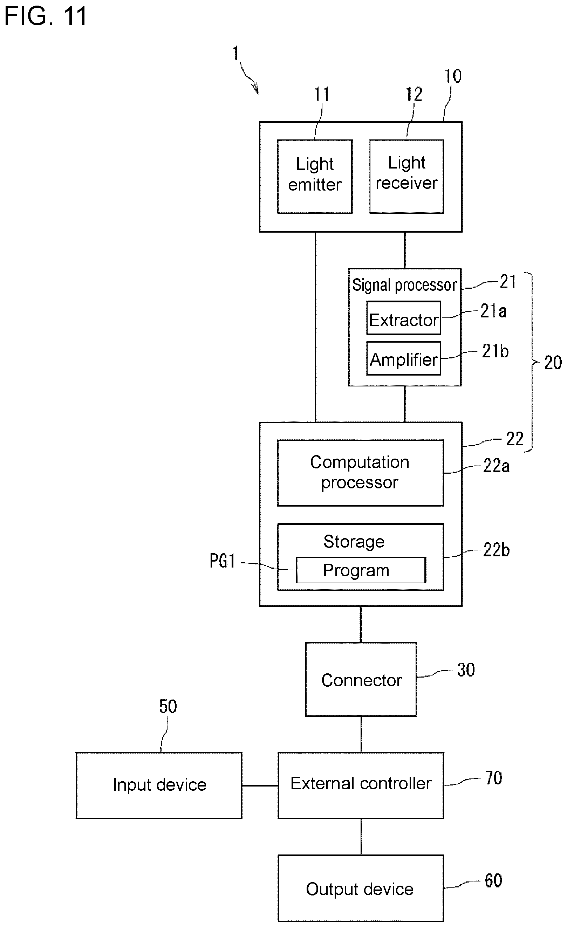

FIG. 11 is a schematic block diagram of a measurement device according to a third embodiment.

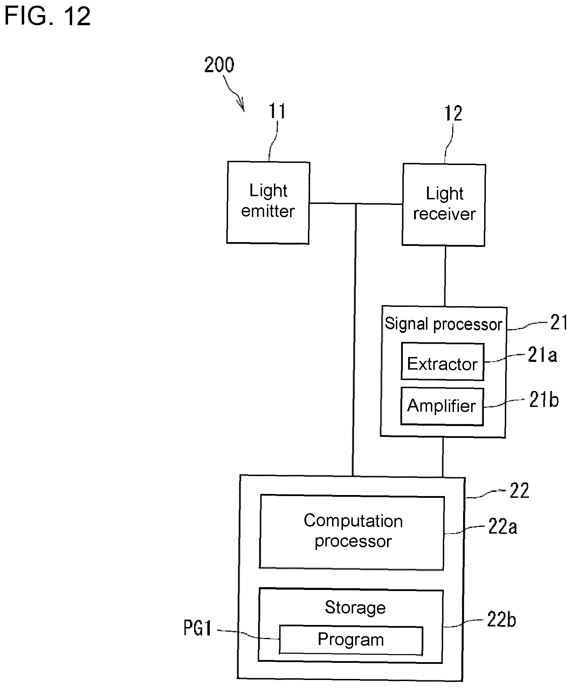

FIG. 12 is a schematic block diagram of a measurement system according to a fourth embodiment.

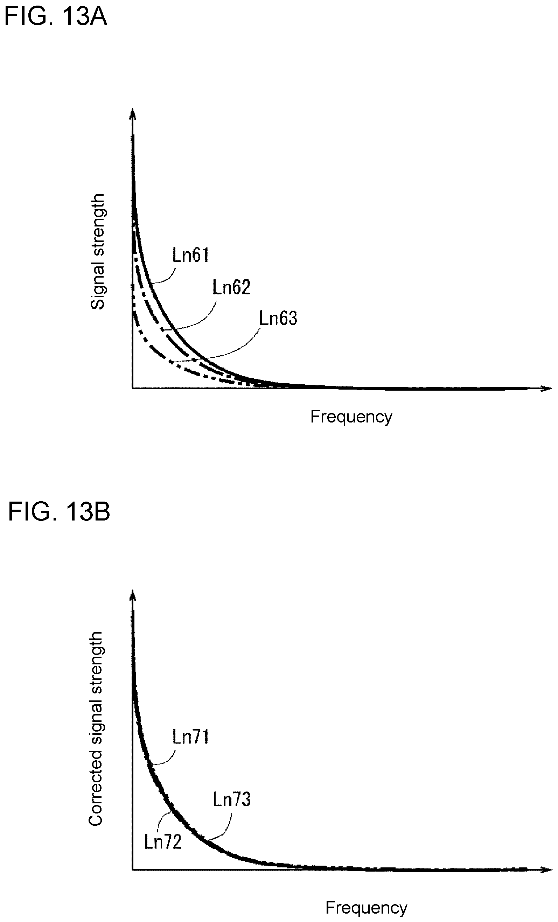

FIG. 13A is a graph for a flow quantitative value with a reference value Q0 showing a curve Ln61 indicating an example frequency spectrum calculated for a particle concentration in a fluid of a first concentration, a curve Ln62 indicating an example frequency spectrum calculated for a particle concentration in a fluid of a second concentration lower than the first concentration, and a curve Ln63 indicating an example frequency spectrum calculated for a particle concentration in a fluid of a third concentration lower than the second concentration, and FIG. 13B is a graph for a flow quantitative value with a reference value Q0 showing a curve Ln71 as an example corrected frequency spectrum calculated for a particle concentration in a fluid of a first concentration, a curve Ln72 as an example corrected frequency spectrum calculated for a particle concentration in a fluid of a second concentration lower than the first concentration, and a curve Ln73 as an example corrected frequency spectrum calculated for a particle concentration in a fluid of a third concentration lower than the second concentration.

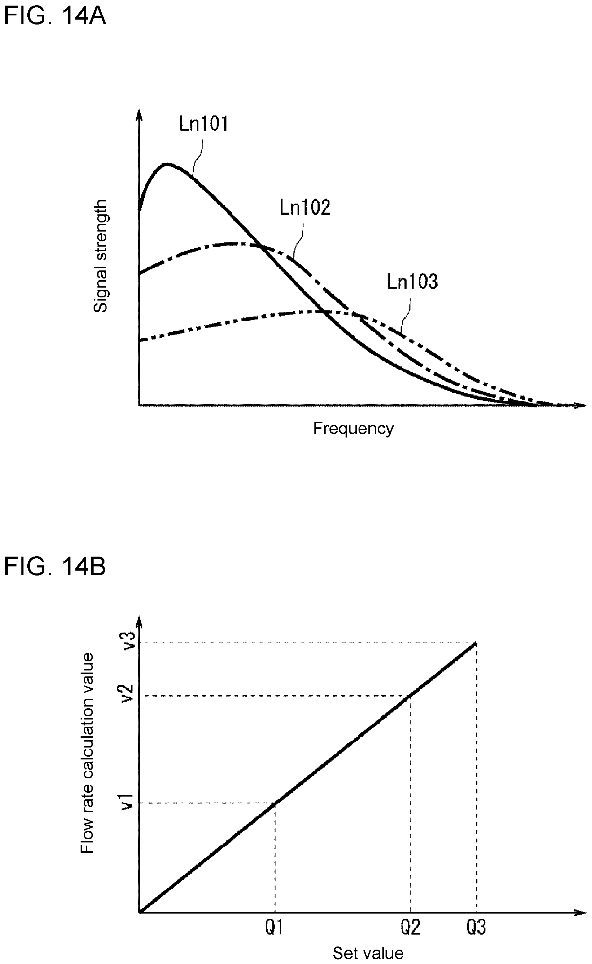

FIG. 14A is a graph for a laser beam with first intensity showing a curve Ln101 indicating an example frequency spectrum of coherent light from an illumination target through which a fluid having a flow rate set value of a relatively small value Q1 flows, a curve Ln102 indicating an example frequency spectrum of coherent light from an illumination target through which a fluid having a flow rate set value of a relatively intermediate value Q2 flows, and a curve Ln103 indicating an example frequency spectrum of coherent light from an illumination target through which a fluid having a flow rate set value of a relatively large value Q3 flows, and FIG. 14B is a graph for a laser beam with first intensity showing an example relationship between a flow rate set value and a flow rate calculation value.

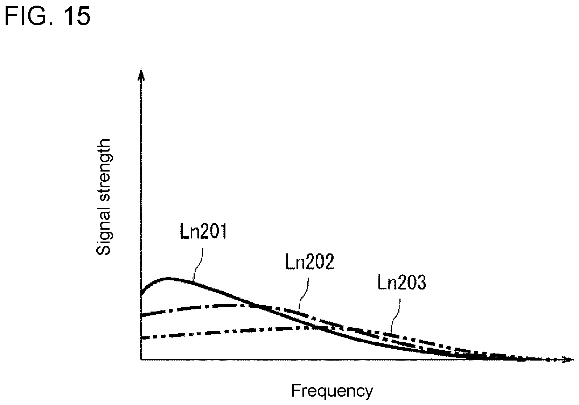

FIG. 15 is a graph for a laser beam with second intensity lower than the first intensity showing a curve Ln201 indicating an example frequency spectrum of coherent light from an illumination target through which a fluid having a flow rate set value of a relatively small value Q1 flows, a curve Ln202 indicating an example frequency spectrum of coherent light from an illumination target through which a fluid having a flow rate set value of a relatively intermediate value Q2 flows, and a curve Ln203 indicating an example frequency spectrum of coherent light from an illumination target through which a fluid having a flow rate set value of a relatively large value Q3 flows.

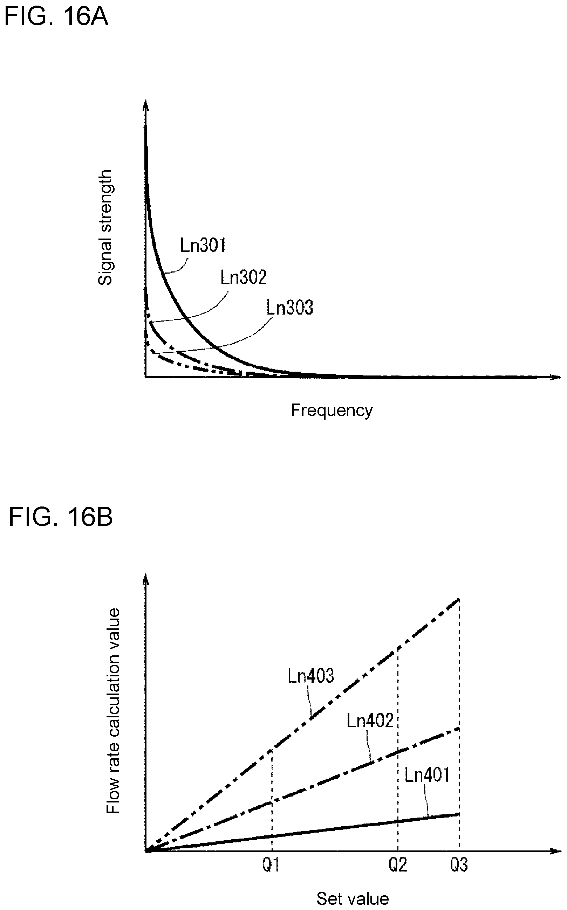

FIG. 16A is a graph for a flow rate set value with a reference value Q0 showing a curve Ln301 indicating an example frequency spectrum calculated for a laser beam with first intensity, a curve Ln302 indicating an example frequency spectrum calculated for a laser beam with second intensity lower than the first intensity, and a curve Ln303 indicating an example frequency spectrum calculated for a laser beam with third intensity lower than the second intensity, and FIG. 16B is a graph showing a line Ln401 indicating an example relationship between a flow rate set value and a flow rate calculation value for a laser beam with first intensity, a line Ln402 indicating an example relationship between a flow rate set value and a flow rate calculation value for a laser beam with second intensity lower than the first intensity, and a line Ln403 indicating an example relationship between a flow rate set value and a flow rate calculation value for a laser beam with third intensity lower than the second intensity.

## DETAILED DESCRIPTION

A device for measuring at least one of a flow rate or a flow velocity of a fluid using an optical method with, for example, a laser blood flowmeter is known as an example of a measurement device that quantitatively measures the flow state of a fluid. This laser blood flowmeter can calculate the blood flow rate of a living body based on, for example, changes in the wavelength of a laser beam, from a laser as a light-emitting device, with which the living body is illuminated due to a Doppler shift resulting from the laser beam scattered.

More specifically, in response to a living body illuminated with a laser beam with a frequency fo, the laser beam incident on the blood flowing through blood vessels (moving objects such as blood cells serving as scatterers) scatters, and the laser beam incident on other fixed tissues (including skin tissue and tissue forming the blood vessels) scatters. Such laser beams form scattered light. The diameter of the blood cells ranges from, for example, several micrometers ($\mu$m) to about 10 $\mu$m. Compared with the frequency fo of the scattered light caused by the other fixed tissues, a frequency f of the scattered light caused by the blood cells serving as scatterers is changed by $\Delta$f to a frequency fo + $\Delta$f by a Doppler shift corresponding to the movement speed of, for example, the blood cells serving as scatterers. This modulated frequency $\Delta$f is expressed with Formula 1 where the velocity of the blood flow is denoted with V, the angle of incidence of a laser beam on the fluid is denoted with $\theta$, and the wavelength of the laser beam is denoted with $\lambda$.

$$\Delta f = (2V \times \cos\theta)/\lambda \qquad (1)$$

Mutual interference between the scattered light with the frequency fo caused by the fixed tissues and the scattered light with the frequency fo + Δf caused by the moving blood cells enables observation of a difference frequency Δf in the form of an optical beat (beat). In other words, a signal (light receiving signal) obtained by receiving these two types of scattered light contains a component of a signal (also referred to as an optical beat signal) corresponding to the optical beat caused by mutual interference of these two types of scattered light.

The difference frequency Δf corresponding to the frequency of the optical beat is far lower than the frequency f of the original light. For example, the original light with a wavelength of 780 nm has a frequency of about 400 terahertz (THz), which exceeds the response speed detectable by a normal photodetector. In contrast, although depending on the movement speed of the blood cells, the frequency Δf of the optical beat (also referred to as an optical beat frequency) is, for example, within the range of about several kilohertz (kHz) to about several tens of kHz and is thus within a frequency range fully responsive and detectable by a normal photodetector. Thus, a signal (light receiving signal) obtained by the photodetector receiving the scattered light with the frequency fo scattered by the fixed tissues and the scattered light with the frequency fo + Δf scattered by the moving blood cells indicates a wave form obtained by superimposing an intensity modulated signal with the optical beat frequency Δf on a direct-current (DC) component signal (DC signal). Then, the optical beat signal with the frequency Δf is analyzed to calculate the blood flow rate.

For example, a frequency spectrum P(f) for a light receiving signal detected by the photodetector is first calculated using a computation such as a fast Fourier transform (FFT). Subsequently, the frequency spectrum P(f) is weighted with the frequency f to calculate a weighted frequency spectrum P(f) × f. Then, the weighted frequency spectrum P(f) × f is integrated within a predetermined frequency range to calculate a first calculation value ($\int\{P(f) \times f\}df$). Subsequently, as shown in Formula 2 below, the first calculation value ($\int\{P(f) \times f\}df$) is divided by a second calculation value ($\int P(f)df$) calculated by integrating the frequency spectrum P(f) within the predetermined frequency range to calculate a mean frequency fm at the optical beat frequency Δf.

$$fm = \int\{P(f) \times f\}df/\{\int P(f)df\} \qquad (2)$$

Subsequently, the blood flow rate of a living body is calculated with a predetermined calculation using the mean frequency fm. A predetermined calculation includes, for example, division of the mean frequency fm by the second calculation value ($\int P(f)df$) and multiplication of the resultant by a constant. The value obtained by dividing the mean frequency fm by the second calculation value ($\int P(f)df$) is calculated as a calculation value corresponding to a flow rate (also referred to as a flow rate calculation value).

In this example, a fluid in which light scatterers of about several micrometers are dispersed flows through a transparent tubular body serving as a flow passage, and a flow rate Q of the fluid is measured with a laser blood flowmeter. In this structure, the flow rate (also referred to as a flow rate set value) of the fluid flowing through the flow passage can be set with, for example, a pump. For example, the flow rate set value is increased to Q1, Q2, and Q3 in this order, and the frequency spectrum P(f) for the optical beat signal, the weighted frequency spectrum P(f) × f, the mean frequency fm, and the flow rate calculation value of the fluid are calculated with the laser blood flowmeter for each of the flow rate set values Q1, Q2, and Q3. For the flow rate set value Q1, for example, a mean frequency f1m is calculated based on the frequency spectrum P(f) indicated with a curve Ln101 drawn with a bold solid line in FIG. 14A. For the flow rate set value Q2, a mean frequency f2m is calculated based on the frequency spectrum P(f) indicated with a curve Ln102 drawn with a bold dot-dash line in FIG. 14A. For the flow rate set value Q3, a mean frequency f3m is calculated based on the frequency spectrum P(f) indicated with a curve Ln103 drawn with a bold two-dot chain line in FIG. 14A.

For example, as drawn with a bold solid line in FIG. 14B, if the flow rate set values Q1, Q2, and Q3 and the flow rate calculation values v1, v2, and v3 calculated in the calculation using the respective mean frequencies f1m, f2m, and f3m are proportional to each other, the flow rates of the fluid are correctly calculated by predetermined calculation with the mean frequency fm.

The intensity of the laser beam with which a living body is illuminated may be reduced due to, for example, a temperature rise or aging degradation of the laser. For example, the intensity of the laser beam may be reduced from the first intensity to the second intensity. The reduced intensity of a laser beam uniformly reduces the strength of light receiving signals output from the photodetector. For the flow rate set value Q1, for example, a frequency spectrum P(f) indicated with a curve Ln201 drawn with a bold solid line in FIG. 15 is obtained. For the flow rate set value Q2, for example, a frequency spectrum P(f) indicated with a curve Ln202 drawn with a bold dot-dash line in FIG. 15 is obtained. For the flow rate set value Q3, for example, a frequency spectrum P(f) indicated with a curve Ln203 drawn with a bold two-dot chain line in FIG. 15 is obtained. In the frequency spectra P(f) in FIG. 15, the strength of signals for respective frequencies is uniformly reduced with the reduced intensity of the laser beam, compared with the frequency spectra P(f) in FIG. 14A.

In this example, FIG. 16A shows example results of the frequency spectra P(f) respectively calculated for a first intensity of 1, a second intensity of 0.5, and a third intensity of 0.25 serving as the intensity of a laser beam intentionally emitted from a laser if the flow rate set value is a constant reference value (also referred to as a reference set value) Q0. FIG. 16A shows a curve Ln301 indicating the frequency spectrum P(f) with a bold solid line calculated for a laser beam with the first intensity, a curve Ln302 indicating the frequency spectrum P(f) with a bold dot-dash line calculated for a laser beam with the second intensity, and a curve Ln303 indicating the frequency spectrum P(f) with a bold two-dot chain line calculated for a laser beam with the third intensity. As shown in FIG. 16A, the reduced intensity of a laser beam reduces the strength of signals for respective frequencies in the frequency spectra P(f). This reduced strength of signals for respective frequencies in the frequency spectra P(f) resulting from the reduced intensity of a laser beam varies the proportional relationship between the flow rate set value and the flow rate calculation value for each intensity of a laser beam as shown in FIG. 16B. More specifically, for a laser beam with the first intensity, the flow rate set value and the flow rate calculation value have a proportional relationship indicated with a line Ln401 drawn with a bold solid line in FIG. 16B. For a laser beam with the second intensity, the flow rate set value and the flow rate calculation value have a proportional relationship indicated with a line Ln402 drawn with a bold dot-dash line in FIG. 16B. For a laser beam with the third intensity, the flow rate set value and the flow rate calculation value have a proportional relationship indicated with a line Ln403 drawn with a bold two-dot chain line in FIG. 16B. Thus, for example, although the flow rate Q of a fluid is calculated from the flow rate calculation value, different flow rates Q of the fluid are calculated in accordance with the intensity of the laser beam, and thus the accuracy in measuring the flow rate Q may be reduced.

Factors for uniformly reducing the strength of light receiving signals output from a photodetector (also referred to as strength reduction factors) are not limited to the reduced intensity of light (also referred to as illumination light) such as a laser beam from a light-emitting device for illuminating a living body. Examples of other factors for reducing the signal strength include the thickness, the inner diameter, and the material of a tubular body defining the flow passage, the particle concentration and light absorptivity in a fluid, and the positional or orientational relationship between the light-emitting device, the tubular body, and the photodetector.

The issues described above occur not only to the measurement device that measures the flow rate of a fluid, but also are common in general measurement devices that measure quantitative values on the flow state of a fluid including at least one of a flow rate or a flow velocity of the fluid.

The inventors of the present disclosure have created a technique of improving measurement accuracy of a measurement device that quantitatively measures the flow state of a fluid.

First to sixth embodiments will now be described below with reference to the drawings. Throughout the drawings, the components having the same structures and functions are given the same reference numerals, and will not be described repeatedly. The drawings are schematic.

1. First Embodiment

1-1. Measurement Device

As shown in FIGs. 1 and 2, a measurement device 1 according to a first embodiment can quantitatively measure, for example, the flow state of a fluid 2b that flows through an internal space 2i of an object (also referred to as a flow passage component) 2a defining a flow passage. The flow passage component 2a may include, for example, a tubular object (also referred to as a tubular body) such as a blood vessel in a living body or pipes in various devices. The quantitative values (also referred to as quantitative values or flow quantitative values) Vq on the flow state of the fluid 2b may include, for example, at least one of the flow rate or the flow velocity. The flow rate is the quantity of a fluid at which the fluid passes through a flow passage per unit time. The quantity of the fluid may be expressed in, for example, a volume or a mass. The flow velocity is the velocity of the fluid flowing through the flow passage. The flow velocity may be expressed with a distance by which the fluid flows per unit time.

The measurement device 1 according to the first embodiment can quantitatively measure the flow state of the fluid 2b with, for example, the Doppler effect for light. For example, if light with which the fluid 2b is illuminated is scattered by the fluid 2b, the Doppler effect corresponding to the flow of the fluid 2b causes a shift (also referred to as a Doppler shift) of the frequency of light corresponding to the movement speed of the fluid 2b. The measurement device 1 according to the first embodiment can measure the flow quantitative value Vq on the flow state of the fluid 2b with this Doppler shift. The components of the measurement device 1 described later can be manufactured with any known methods as appropriate.

Examples of the fluid 2b serving as a target (also referred to as a measurement target) having its flow state quantitatively measured include the fluid 2b that scatters light, a fluid that allows a substance that scatters light (also referred to as a scatter substance), and an object that scatters light (also referred to as a scatterer) to flow through the fluid. More specifically, examples of the fluid 2b serving as a measurement target include water, blood, printer ink, and gas containing a scatterer such as powder. If a scatter substance or a scatterer flows with the fluid, the flow rate of the scatter substance or the scatterer may be used as the flow rate of a fluid, or the flow velocity of the scatter substance or the scatterer may be used as the flow velocity of the fluid.

As shown in FIGs. 1 and 2, the measurement device 1 according to the first embodiment includes, for example, a sensor 10 and a controller 20. The measurement device 1 also includes, for example, a connector 30.

The sensor 10 includes, for example, a light emitter 11 and a light receiver 12.

The light emitter 11 can illuminate, with light (also referred to as illumination light) L1, an object (also referred to as an illumination target) 2 having the internal space 2i through which the fluid 2b flows. The illumination target 2 includes at least an object (flow passage component) 2a defining a flow passage of a tubular body, and a fluid 2b flowing through the flow passage. Examples of the illumination light L1 include light having a predetermined wavelength suitable for the fluid 2b serving as a measurement target. For example, if the fluid 2b is blood, the illumination light L1 having a wavelength set to about 600 to 900 nanometers (nm) is used. For example, if the fluid 2b is printer ink, the illumination target 2 is illuminated with the light having a wavelength set to about 700 to 1000 nm. A semiconductor laser device such as a vertical-cavity surface-emitting laser (VCSEL) is used as an example of the light emitter 11. In this case, the intensity of the illumination light L1 may be reduced due to, for example, a temperature rise or aging degradation of the semiconductor laser device.

The light receiver 12 can receive coherent light L2 including light scattered by the illumination target 2 in the illumination light L1. For example, the light receiver 12 can convert the received light to an electric signal corresponding to the light intensity. In other words, the light receiver 12 can receive the coherent light L2 including light scattered by the illumination target 2, and output a signal corresponding to the intensity of the coherent light L2. The coherent light L2 that can be received by the light receiver 12 includes coherent light, in the light scattered by the illumination target 2, caused by scattered light without a Doppler shift from an object stationary around the fluid 2b (also referred to as a stationary object) and scattered light with a Doppler shift with a shift amount of $\Delta f$ from the fluid 2b. For blood flowing through a blood vessel serving as an example of the fluid 2b, the stationary object includes an object (flow passage component) 2a such as the skin or blood vessel. For ink flowing through a pipe serving as an example of the fluid 2b, the stationary object includes an object (flow passage component) 2a defining a flow passage for the fluid 2b such as a pipe. In this case, the pipe may be formed from, for example, a translucent material. Examples of the translucent material include glass and polymer resin.

A change in the intensity of the coherent light L2 with time (also referred to as a temporal change) can indicate a beat of the frequency corresponding to a difference (also referred to as a difference frequency) $\Delta f$ between the frequency of scattered light without a Doppler shift and the frequency of scattered light with a Doppler shift. Thus, a signal output from the light receiver 12 and corresponding to the intensity of the coherent light L2 can contain a component of a signal corresponding to the beat (also referred to as a beat signal or an optical beat signal) with respect to the temporal change in the intensity of the coherent light L2. A device that can follow the beat (also referred to as having time resolution) with respect to the temporal change in the intensity of the coherent light L2 is usable as an example of the light receiver 12. The wavelength of light that can be received by the light receiver 12 can be set in accordance with the measurement conditions such as the wavelength of the illumination light L1 and the velocity range of the fluid 2b. Examples of the light receiver 12 include various photodiodes including a silicon (Si) photodiode, a gallium arsenide (GaAs) photodiode, an indium gallium arsenide (InGaAs) photodiode, and a germanium (Ge) photodiode.

The sensor 10 may also include a package 13. The package 13 accommodates the light emitter 11 and the light receiver 12. In the example in FIG. 2, the measurement device 1 includes a substrate (also referred to as a mounting board) 1s that receives the sensor 10, the controller 20, and the connector 30. Examples of the mounting board 1s include a printed circuit board. The package 13 in the sensor 10 is located on the mounting board 1s. The mounting board 1s electrically connects, for example, the sensor 10 to the controller 20 and the controller 20 to the connector 30.

The package 13 has, for example, a cubic or rectangular parallelepiped external shape. The package 13 includes, for example, a first recess R1 and a second recess R2 open upward. The first recess R1 receives the light emitter 11. The second recess R2 receives the light receiver 12. The illumination light L1 emitted from the light emitter 11 is, for example, applied to the illumination target 2 through the opening of the first recess R1. The coherent light L2 from the illumination target 2 is, for example, received by the light receiver 12 through the opening of the second recess R2. The package 13 may be, for example, a multilayered wiring board formed from ceramic or organic materials. Examples of the ceramic material include sintered aluminum oxide and sintered mullite. Examples of the organic material include an epoxy resin and a polyimide resin.

As shown in FIG. 2, a translucent cover 14 may be located to cover the openings of the first recess R1 and the second recess R2 in the package 13. This structure can hermetically seal the light emitter 11 in the first recess R1 in the package 13, and the light receiver 12 in the second recess R2 in the package 13. The cover 14 may be, for example, a glass plate.

The controller 20 can control, for example, the measurement device 1. The controller 20 includes, for example, multiple electronic components including an active element such as a transistor or a diode and a passive element such as a capacitor. The connector 30 can electrically connect, for example, the controller 20 to external devices. For example, multiple electronic components may be integrated to form one or more integrated circuits (ICs) or large-scale integration circuits (LSIs), or multiple ICs or LSIs may be further integrated to form various functional units including the controller 20 and the connector 30. Multiple electronic components forming the controller 20 and the connector 30 are mounted on the mounting board 1s. The package 13 is thus electrically connected to the controller 20, and the controller 20 is electrically connected to the connector 30.

The controller 20 includes, for example, a signal processor 21 and an information processor 22.

The signal processor 21 can perform, for example, various processes on an electric signal received from the light receiver 12. Examples of the various processes may include conversion of an electric signal into a voltage, separation of an electric signal into an alternating current (AC) component and a direct current (DC) component, amplification of the strength of an electric signal, and conversion of an analog signal to a digital signal. Thus, the signal processor 21 functions as, for example, a unit (also referred to as an extractor) 21a that extracts, from a signal output from the light receiver 12, a DC component in a signal at the temporal change in the signal strength (also referred to as signal strength). The signal processor 21 may also function as a unit (also referred to as an amplifier) 21b that can, for example, amplify a signal. For example, the extractor 21a may separate the electric signal output from the light receiver 12 into DC and AC components, and then the amplifier 21b may amplify the AC component signal (also referred to as an AC signal). The various processes performed by the signal processor 21 may include conversion of an electric signal to a voltage, separation of an electric signal into AC and DC components (also referred to as AC-DC separation), amplification of the AC signal, and conversion of an analog signal to a digital signal.

The signal processor 21 may include a circuit such as a current-voltage conversion circuit (I-V conversion circuit), an AC-DC separation circuit (AC-DC decoupling circuit) serving as the extractor 21a, an AC amplifier circuit serving as the amplifier 21b, and an analog-to-digital conversion circuit (AD conversion circuit). In this example, the extractor 21a can extract, from the signal output from the light receiver 12, AC and DC components in the signal at the temporal change in the signal strength. For example, after causing the amplifier 21b to amplify the signal output from the light receiver 12 and causing the extractor 21a to separate the electric signal into AC and DC components, the signal processor 21 may extract the AC and DC components. The signal processor 21 can thus perform processing such as AC-DC separation, amplification, and AD conversion on an analog electric signal received from the light receiver 12, and then output a digital signal to the information processor 22.

The information processor 22 includes, for example, a computation processor 22a and a storage 22b.

The computation processor 22a includes, for example, a processor as an electric circuit. The processor may include, for example, one or more processors, a controller, a microprocessor, a microcontroller, an application-specific integrated circuit (ASIC), a digital signal processor, a programmable logic device, a combination of any of these devices or components, or a combination of any other known devices or components.

The storage 22b includes, for example, a random-access memory (RAM) and a read-only memory (ROM). The storage 22b stores, for example, firmware containing a program PG1. The computation processor 22a can perform, for example, computation or processing on one or more pieces of data in accordance with the firmware stored in the storage 22b. In other words, for example, the computation processor 22a executing the program PG1 enables implementation of the various functions of the measurement device 1. Thus, the information processor 22 can control, for example, the operation of the light emitter 11 and the light receiver 12.

The frequency and the signal strength of an electric signal output from the light receiver 12 depend on the Doppler effect for light. Thus, the frequency spectrum P(f) showing the relationship between the frequency and the strength of the electric signal changes in accordance with the flow quantitative value (flow rate or flow velocity) Vq of the fluid 2b. Thus, the information processor 22 can perform, for example, computation to quantitatively measure the flow state of the fluid 2b based on the electric signal output from the light receiver 12 and then processed by the signal processor 21 with the computation processor 22a.

The computation processor 22a can calculate, for example, a power spectrum (also referred to as a frequency spectrum) P(f) indicating the distribution of the signal strength for each frequency at a temporal change in the signal strength of the signal output from the light receiver 12. In other words, the computation processor 22a can calculate, for example, the frequency spectrum P(f) for the signal output from the light receiver 12 at the temporal change in the signal strength. More specifically, the computation processor 22a can calculate the frequency spectrum P(f) with respect to a change in the signal strength over time (temporal change) for an AC signal obtained by AC-DC separation and amplification with the signal processor 21 that processes the signals output from the light receiver 12. The frequency spectrum P(f) is obtained by performing an analysis with a computation such as a Fourier transform on a temporal change in the strength of an AC signal output from the signal processor 21. The frequency range in the frequency spectrum P(f) can be set based on, for example, a sampling rate in an AD conversion circuit.

For the fluid 2b having the flow quantitative value (flow rate or flow velocity) Vq of the relatively small value Vq1, for example, the computation processor 22a can calculate the frequency spectrum P(f) indicated with the curve Ln1 drawn with a bold solid line in FIG. 3A. For the fluid 2b having the flow quantitative value (flow rate or flow velocity) Vq of the relatively intermediate value Vq2, for example, the computation processor 22a can calculate the frequency spectrum P(f) indicated with the curve Ln2 drawn with a bold dot-dash line in FIG. 3A. For the fluid 2b having the flow quantitative value (flow rate or flow velocity) Vq of the relatively large value Vq3, for example, the computation processor 22a can calculate the frequency spectrum P(f) indicated with the curve Ln3 drawn with a bold two-dot chain line in FIG. 3A. In response to an increase in the flow quantitative value Vq of the fluid 2b, the signal strength gradually increases or decreases with a change in the frequency to form the shape of the frequency spectrum P(f) as shown in FIG. 3A. The computation processor 22a can obtain a DC signal obtained by, for example, the signal processor 21 performing AC-DC separation and amplification on the signal output from the light receiver 12. For example, the computation processor 22a can obtain a signal with a DC component indicated with a line Ln4 drawn with a bold solid line shown in FIG. 3B. The computation processor 22a can obtain, for example, a mean value of the signal strength of DC signals within a predetermined time or the signal strength of a DC signal at predetermined timing as a signal strength Pd of a DC component. In this example, the computation processor 22a can obtain, for the illumination light L1 with the first intensity, the frequency spectrum P(f) in FIG. 3A and the signal strength Pd of the DC component in FIG. 3B.

In this example, the illumination light L1 has the second intensity lower than the first intensity. In this case, if the fluid 2b has the flow quantitative value (flow rate or flow velocity) Vq of a relatively small value Vq1, for example, the frequency spectrum P(f) calculated by the computation processor 22a is indicated with the curve Ln11 drawn with a bold solid line in FIG. 4A. If, for example, the fluid 2b has the flow quantitative value (flow rate or flow velocity) Vq of a relatively intermediate value Vq2, the frequency spectrum P(f) calculated by the computation processor 22a is indicated with the curve Ln12 drawn with a bold dot-dash line in FIG. 4A. If, for example, the fluid 2b has the flow quantitative value (flow rate or flow velocity) Vq of a relatively large value Vq3, the frequency spectrum P(f) calculated by the computation processor 22a is indicated with the curve Ln13 drawn with a bold two-dot chain line in FIG. 4A. The DC component signal obtained by the computation processor 22a is, for example, indicated with a line Ln14 drawn with a bold solid line in FIG. 4B.

For the same flow quantitative value Vq of the fluid 2b, the intensity of the illumination light L1 reduced from the first intensity to the second intensity reduces the intensity of coherent light L2 received by the light receiver 12. As shown in, for example, FIGs. 3A and 4A, the signal strength in the frequency spectrum P(f) is uniformly reduced. In this example, as shown in FIGs. 3B and 4B, the signal strength Pd of the DC component obtained by the computation processor 22a is also reduced as in the signal strength of the frequency spectrum P(f).

Calculation of Flow Calculation Value

The computation processor 22a can perform a process including correction using a value (also referred to as a D value) Vd of the signal strength Pd of the DC component, for example, on the frequency spectrum P(f) for the AC component of the signal output from the light receiver 12, and calculate a calculation value (also referred to as a flow calculation value) F of the flow state of the fluid 2b. The flow state may include, for example, at least one of the flow rate or the flow velocity.

In the first embodiment, the computation processor 22a first calculates the frequency spectrum (also referred to as a first frequency spectrum) P1(f) for the AC component of the signal output from the light receiver 12, and corrects, with a value (D value) Vd of the signal strength Pd of the DC component, the signal strength for each frequency in the first frequency spectrum P1(f). Thus, the corrected frequency spectrum (also referred to as a second frequency spectrum) P2(f) is calculated. The computation processor 22a calculates a calculation value (flow calculation value) F of the flow state of the fluid 2b based on the second frequency spectrum P2(f).

As an example of correction using the value (D value) Vd of the signal strength Pd of the DC component, division using the D value Vd is used. In this example, the division using the D value Vd can cancel any uniformly reduced strength of signals output from the light receiver 12 with the DC component reduced together with the uniformly reduced signal strength. More specifically, examples of correction using the D value Vd include division of the first frequency spectrum P1(f) by the D value Vd. Examples of the D value Vd include the signal strength Pd of the DC component raised to the m-th (m is a predetermined positive number) power. In this case, Formula 3 below holds. For example, 1.3 is used as an exponent m whereas the signal strength Pd is used as the base.

$$P2(f) = P1(f)/(Pd)^{m} \qquad (3)$$

In Formula 3, at least one of the denominator or the numerator on the right side or the entire right side may undergo one or more calculations such as multiplication by a coefficient, exponentiation, and addition or subtraction of a constant.

For example, the flow calculation value F based on the second frequency spectrum P2(f) is calculated in the manner described below. The second frequency spectrum P2(f) is weighted with the frequency f to calculate a weighted frequency spectrum (also referred to as a third frequency spectrum) P2(f) $\times$ f. Then, the third frequency spectrum P2(f) $\times$ f is integrated within a predetermined frequency range to calculate a first integral ($\int${P2(f) $\times$ f}df). The second frequency spectrum P2(f) is integrated within a predetermined frequency range to calculate a second integral ($\int$P2(f)df). Subsequently, the first integral is divided by the second integral to calculate a value corresponding to a mean frequency fm in the difference frequency $\Delta$f.This value is also divided by the second integral ($\int$P2(f)df) to calculate a flow calculation value F. In this case, Formula 4 below holds. The second division with the second integral ($\int$P2(f)df) is performed to, for example, correct the attenuation of the amplification factor with respect to the increase in the frequency in the amplifier 21b.

$$F = \int\{P2(f) \times f\}df/[\int P2(f)df]^2 \qquad (4)$$

In Formula 4, for example, at least one of the denominator or the numerator on the right side or the entire right side may undergo one or more calculations such as multiplication by a coefficient, exponentiation, and addition or subtraction of a constant. In this case, instead of the second division with the second integral ($\int$P2(f)df), for example, division with a specific value of the signal strength of the second frequency spectrum P2(f) may be performed. The specific value of the signal strength is, for example, a maximum value of the signal strength, the signal strength in a specific frequency, or the signal strength in an intermediate frequency. Examples of the intermediate frequency can include a boundary frequency at which an integral of the strength of lower frequencies and an integral of the strength of higher frequencies in the second frequency spectrum P2(f) have a predetermined ratio. The predetermined ratio may be set to 1:1.

In this example, the measurement device 1 sets the intensity of the illumination light L1 to the first intensity of 1, the intensity half the first intensity (also referred to as the second intensity) of 0.5, or the intensity half the second intensity (also referred to as the third intensity) of 0.25, using the quantitative value (flow quantitative value) Vq on the flow state of the fluid 2b flowing through a transparent tube serving as the flow passage component 2a set to a predetermined value with, for example, a pump. In this example, for the illumination light L1 with the first intensity, the measurement device 1 can obtain the first frequency spectrum P1(f) indicated with the curve Ln21 drawn with a bold solid line in FIG. 5A. For the illumination light L1 with the second intensity, the measurement device 1 can obtain the first frequency spectrum P1(f) indicated with the curve Ln22 drawn with a bold dot-dash line in FIG. 5A. For the illumination light L1 with the third intensity, the measurement device 1 can obtain the first frequency spectrum P1(f) indicated with the curve Ln23 drawn with a bold two-dot chain line in FIG. 5A. As shown in FIG. 5A, the strength of the first frequency spectrum P1(f) is reduced with the reduced intensity of the illumination light L1.

In this example, a reference flow calculation value Fo is calculated without performing correction using the D value Vd with the flow quantitative value Vq being varied. In this case, the reference flow calculation value Fo is calculated in accordance with Formula 5 below.

$$Fo = \int\{P1(f) \times f\}df/[\int P1(f)df]^2 \qquad (5)$$

Also in Formula 5, for example, at least one of the denominator or the numerator on the right side or the entire right side may undergo one or more calculations such as multiplication by a coefficient, exponentiation, and addition or subtraction of a constant. In Formula 5, instead of the second division using the integral $\int$P1(f), for example, division with a specific value of the signal strength of the frequency spectrum P1(f) may be performed.

In this example, for the illumination light L1 with the first intensity, the flow quantitative value and the reference flow calculation value Fo have a relationship indicated with the line Ln31 drawn with a bold solid line in FIG. 5B. For the illumination light L1 with the second intensity, for example, the flow quantitative value and the reference flow calculation value Fo have a relationship indicated with the line Ln32 drawn with a bold dot-dash line in FIG. 5B. For the illumination light L1 with the third intensity, for example, the flow quantitative value and the reference flow calculation value Fo have a relationship indicated with the line Ln33 drawn with a bold two-dot chain line in FIG. 5B. As shown in FIG. 5B, the proportional relationship between the flow quantitative value and the reference flow calculation value Fo differs depending on the intensity of the illumination light L1.

In contrast, for example, the measurement device 1 according to the first embodiment corrects the first frequency spectrum P1(f) with the D value Vd to calculate the corrected frequency spectrum (second frequency spectrum) P2(f). In this case, as shown in FIG. 6A, the second frequency spectra P2(f) are almost the same independently of the intensity of the illumination light L1 unlike the first frequency spectra P1(f) shown in FIG. 5A. For example, the curve Ln41 drawn with a bold solid line in FIG. 6A indicates the second frequency spectrum P2(f) for the illumination light L1 with the first intensity. For example, the curve Ln42 drawn with a bold dot-dash line in FIG. 6A indicates the second frequency spectrum P2(f) for the illumination light L1 with the second intensity. For example, the curve Ln43 drawn with a bold two-dot chain line in FIG. 6A indicates the second frequency spectrum P2(f) for the illumination light L1 with the third intensity.

As shown in FIG. 6B, compared with the proportional relationship between the flow quantitative value and the reference flow calculation value Fo shown in FIG. 5B, the flow calculation value F calculated in accordance with Formula 4 indicates the proportional relationships between the flow quantitative value and the flow calculation value F that are almost the same independently of the intensity of the illumination light L1. For example, the line Ln51 drawn with a bold solid line in FIG. 6B indicates the relationship between the flow quantitative value and the flow calculation value F for the illumination light L1 with the first intensity. For example, the line Ln52 drawn with a bold dot-dash line in FIG. 6B indicates the relationship between the flow quantitative value and the flow calculation value F for the illumination light L1 with the second intensity. For example, the line Ln53 drawn with a bold two-dot chain line in FIG. 6B indicates the relationship between the flow quantitative value and the flow calculation value F for the illumination light L1 with the third intensity.

A signal output from the light receiver 12 having the uniformly reduced strength is corrected using the D value Vd of the strength of the DC component of the signal output from the light receiver 12 to reduce variations in the relationship between the flow calculation value F and the actual flow state of the fluid 2b.

Calculation of Flow Quantitative Value

The computation processor 22a can calculate a quantitative value (flow quantitative value) Vq indicating the flow state of the fluid 2b based on, for example, the flow calculation value F calculated as described above. For example, the computation processor 22a can calculate the quantitative value (flow quantitative value) Vq on the flow of the fluid 2b based on the flow calculation value F and prepared calibration data (also referred to as a calibration curve). If, for example, the calibration data on the flow rate of the fluid 2b is prepared in advance, the flow rate of the fluid 2b can be calculated based on the flow calculation value F and the calibration curve of the flow rate serving as the flow quantitative value Vq. If, for example, the calibration data on the flow velocity of the fluid 2b is prepared in advance, the flow velocity of the fluid 2b can be calculated based on the flow calculation value F and the calibration curve of the flow velocity serving as the flow quantitative value Vq. Thus, at least one of the flow rate or the flow velocity of the fluid 2b can be calculated. As described above, for example, any uniformly reduced strength of the signal output from the light receiver 12 is less likely to change the relationship between the flow calculation value F and the actual flow state of the fluid 2b. Thus, the measurement device 1 can have higher measurement accuracy.

For example, the calibration data may be stored in the storage 22b or other storage in advance before the flow quantitative value Vq of the fluid 2b is measured. The calibration data may be stored in the form of, for example, a functional formula or a table.

The calibration data can be prepared by, for example, the measurement device 1 calculating the flow calculation value F of the fluid 2b, as a measurement target, flowing through the flow passage component 2a at a known flow quantitative value Vq. The calculation of the flow calculation value F performed by the measurement device 1 includes the light emitter 11 illuminating the illumination target 2 with the illumination light L1, the light receiver 12 receiving the coherent light L2 including light scattered by the illumination target 2, and the computation processor 22a calculating the flow calculation value F. For example, the measurement device 1 calculates the flow calculation value F of the fluid 2b flowing through the flow passage component 2a at a known flow quantitative value Vq, and derives calibration data based on the relationship between the known flow quantitative value Vq and the flow calculation value F. More specifically, for example, an operation expression (calibration curve) including the flow calculation value F as a parameter is derived as calibration data.

For example, the calibration curve is written by Formula 6 including the flow quantitative value Vq denoted with y, the flow calculation value F denoted with x, a coefficient a, a coefficient b, and a constant c.

$$y = a \times x^2 + b \times x + c \qquad (6)$$

If, for example, a flow calculation value F of the fluid 2b flowing through the flow passage component 2a at the flow quantitative value Vq of a known value y1 is calculated as a value x1, a flow calculation value F of the fluid 2b flowing through the flow passage component 2a at the flow quantitative value Vq of a known value y2 is calculated as a value x2, and a flow calculation value F of the fluid 2b flowing through the flow passage component 2a at the flow quantitative value Vq of a known value y3 is calculated as a value x3, Formulas 7, 8, and 9 below are obtained.

$$y1 = a \times x1^2 + b \times x1 + c \qquad (7)$$

$$y2 = a \times x2^2 + b \times x2 + c \qquad (8)$$

$$y3 = a \times x3^2 + b \times x3 + c \qquad (9)$$

The coefficients a and b and the constant c are calculated using Formulas 7, 8, and 9. The calculated coefficients a and b and constant c are substituted into Formula 6 to obtain the calibration data indicating the calibration curve.

The functional formula representing the calibration curve may be, for example, written using a polynomial expression including an n-th order term (n is a natural number greater than or equal to 2), where the flow quantitative value Vq is denoted with y and the flow calculation value F is a variable x. The functional formula representing the calibration curve may include, for example, at least one term selected from the term of logarithm and the term of exponentiation of a variable x serving as the flow calculation value F.

1-2. Operation of Measurement Device

The operation of the measurement device 1 will now be described using an example. FIGs. 7A and 7B are flowcharts showing an example operation of the measurement device 1. The operation can be performed by, for example, the computation processor 22a executing the program PG1 and the controller 20 controlling the operation of the measurement device 1. The flow quantitative value Vq indicating the flow state of the fluid 2b can be calculated by performing steps SP1 to SP4 in FIG. 7A.

Step SP1 in FIG. 7A is a process (also referred to as a first process) in which, while the light emitter 11 illuminates, with light, the illumination target 2 having the internal space 2i through which the fluid 2b flows, the light receiver 12 receives the coherent light L2 including light scattered by the illumination target 2 and outputs a signal corresponding to the intensity of the coherent light L2.

Step SP2 is a process (also referred to as a second process) in which the signal processor 21 processes the signal output from the light receiver 12 in step SP1. For example, the extractor 21a in the signal processor 21 extracts a DC component in the signal output from the light receiver 12 in step SP1 at the temporal change in the signal strength. The DC component is extracted through, for example, the AC-DC separation by separating the signal output from the light receiver 12 into DC and AC components. Instead of performing AC-DC separation on the signal output from the light receiver 12 to extract the DC component, the signal processor 21 may perform other operations on the signal, such as AD conversion and amplification performed using the amplifier 21b. For example, after the extractor 21a separates the electric signal output from the light receiver 12 into the DC and AC components, the amplifier 21b may amplify the AC signal including the AC component. In some embodiments, after the amplifier 21b amplifies a signal output from the light receiver 12, the extractor 21a may separate the electric signal into DC and AC components. The signal resulting from the processing by the signal processor 21 is input into the information processor 22 as appropriate.

Step SP3 is a process (also referred to as a third process) in which the computation processor 22a calculates the flow calculation value F by performing, based on the signal output from the light receiver 12 in step SP1, correction using the value (D value) Vd of the signal strength Pd of the DC component extracted by the extractor 21a in step SP2 and calculation of the frequency spectrum. Step SP3 includes, for example, steps SP31 to SP33 in FIG. 7B performed in the stated order.

In step SP31, the computation processor 22a calculates the distribution (first frequency spectrum) P1(f) of the signal strength for each frequency with respect to the temporal strength change in the signal output from the light receiver 12 in step SP1. In this example, the computation processor 22a calculates the first frequency spectrum P1(f) for the AC signal obtained through the processing performed by the signal processor 21 in step SP2.

In step SP32, the computation processor 22a corrects the signal strength of the first frequency spectrum P1(f) calculated in step SP31 using the D value Vd of the signal strength Pd of the DC component extracted by the extractor 21a in step SP2. In this example, the computation processor 22a performs division using the D value Vd. More specifically, for example, the computation processor 22a divides the first frequency spectrum P1(f) with the signal strength Pd of the DC component raised to the m-th (m is a predetermined positive number) power in accordance with Formula 3. Thus, the computation processor 22a calculates the second frequency spectrum P2(f) as a corrected frequency spectrum.

In step SP33, the computation processor 22a calculates the flow calculation value F based on the second frequency spectrum P2(f) calculated in step SP32. In this example, the computation processor 22a calculates a first

integral ($\int\{P2(f) \times f\}df$) for a third frequency spectrum $P2(f) \times f$ obtained by weighting the second frequency spectrum P2(f) with the frequency f in accordance with Formula 4. The computation processor 22a calculates a second integral ($\int P2(f)df$) for the second frequency spectrum P2(f). In this case, the computation processor 22a divides the first integral ($\int\{P2(f) \times f\}df$) with the second integral ($\int P2(f)df$) to calculate a value corresponding to the mean frequency fm in a difference frequency $\Delta f$. The computation processor 22a further divides this value with the second integral ($\int P2(f)df$) to calculate the flow calculation value F. This calculation may include, for example, one or more calculations such as multiplication by a coefficient, exponentiation, and addition or subtraction of a constant to be performed on each value. Instead of the second division with the second integral, for example, division using a specific value of the signal strength of the second frequency spectrum $\int P2(f)$ may be performed.

In this example, the first frequency spectrum P1(f) is corrected with the D value Vd of the signal strength of the DC component of the signal output from the light receiver 12. Thus, for example, any uniformly reduced strength of the signal output from the light receiver 12 is less likely to change the relationship between the flow calculation value F and the actual flow state of the fluid 2b. Thus, the measurement device 1 can easily have higher measurement accuracy.

In step SP4, the computation processor 22a calculates the flow quantitative value Vq based on the flow calculation value F calculated in step SP3. The flow quantitative value Vq includes at least one of the flow rate or the flow velocity of the fluid 2b.

In some embodiments, step SP3 may include, for example, the processing in steps SP31A to SP33A in FIG. 8 performed sequentially. In this example, the computation processor 22a may correct at least the signal strength of the AC component in the signal output from the light receiver 12 using the value (D value) Vd of the signal strength Pd of the DC component to calculate a frequency spectrum (also referred to as the third frequency spectrum) for the signal strength of the corrected AC component and calculate the flow calculation value F based on the third frequency spectrum.

In step SP31A, the computation processor 22a corrects at least the strength of the AC component included in the signal output from the light receiver 12 in step SP1 using the value (D value) Vd of the signal strength Pd of the DC component extracted by the extractor 21a in step SP2. In this case, for example, the computation processor 22a obtains the corrected AC signal by dividing the AC signal strength obtained in step SP2 with the D value Vd of the signal strength Pd of the DC component obtained in step SP2. The D value Vd is, for example, the signal strength Pd of the DC component raised to the m-th (m is a predetermined positive number) power. This calculation may include, for example, one or more calculations such as multiplication by a coefficient and exponentiation to be performed on each value.

In step SP32A, the computation processor 22a calculates the frequency spectrum (third frequency spectrum) P(f) for the corrected AC signal obtained in step SP31A at the temporal change in the signal strength.

In step SP33A, the computation processor 22a calculates the flow calculation value F for the flow state of the fluid 2b flowing through the internal space 2i of the illumination target 2 based on the third frequency spectrum P(f) calculated in step SP32A. In this example, the computation processor 22a calculates the first integral ($\int\{P(f) \times f\}df$) for a weighted frequency spectrum $P(f) \times f$ by weighting the third frequency spectrum P(f) calculated in step SP32A with the frequency f, and calculates the second integral ($\int P(f)df$) for the third frequency spectrum P(f) calculated in step SP32A. In this case, the computation processor 22a divides the first integral ($\int\{P(f) \times f\}df$) with the second integral ($\int P(f)df$) to calculate a value corresponding to the mean frequency fm in the difference frequency $\Delta f$, and further divides this value with the second integral ($\int P(f)df$) to calculate the flow calculation value F. This calculation may include, for example, one or more calculations such as multiplication by a coefficient, exponentiation, and addition or subtraction of a constant to be performed on each value. Instead of the second division with the second integral, for example, division using a specific value of the signal strength of the third frequency spectrum may be performed.

In this example, the signal output from the light receiver 12 is corrected with the D value Vd of the strength of the DC component of the signal output from the light receiver 12. Thus, any uniformly reduced strength of the signal output from the light receiver 12 is less likely to change the relationship between the flow calculation value F and the actual flow state of the fluid 2b. Thus, the measurement device 1 can easily have higher measurement accuracy.

In some embodiments, step SP3 may include the processing in steps SP31B to SP33B in FIG. 9 performed sequentially. In this example, the computation processor 22a may calculate a frequency spectrum (also referred to as a fourth frequency spectrum) for the signal output from the light receiver 12 at the temporal change in the signal strength, and calculate the flow calculation value F with a computation including correction using the value (D value) Vd of the signal strength Pd of the DC component based on the fourth frequency spectrum.

In step SP31B, the computation processor 22a calculates the frequency spectrum (fourth frequency spectrum) P(f) for the signal output from the light receiver 12 in step SP1 at the temporal change in the signal strength. In this example, the computation processor 22a calculates the fourth frequency spectrum P(f) for the AC signal obtained through the processing performed by the signal processor 21 in step SP2.

In steps SP32B and SP33B, the computation processor 22a performs a computation including correction using the value (D value) Vd of the signal strength Pd of the DC component extracted by the extractor 21a in step SP2 based on the fourth frequency spectrum P(f) calculated in step SP31B to calculate the flow calculation value F.

More specifically, for example, in step SP32B, the computation processor 22a calculates a temporary flow calculation value Fp with the fourth frequency spectrum P(f) calculated in step SP31B. In this example, the computation processor 22a calculates the first integral ($\int\{P(f) \times f\}df$) for the weighted frequency spectrum P(f) $\times$ f obtained by weighting the fourth frequency spectrum P(f) calculated in step SP31B with the frequency f, and calculates the second integral ($\int P(f)df$) for the fourth frequency spectrum P(f) calculated in step SP31B. In this case, the computation processor 22a divides the first integral ($\int\{P(f) \times f\}df$) with the second integral ($\int P(f)df$) to calculate a value corresponding to a temporary mean frequency fmp in the difference frequency $\Delta f$, and further divides this value with the second integral ($\int P(f)df$) to calculate the temporary flow calculation value Fp. This calculation may include, for example, one or more calculations such as multiplication by a coefficient, exponentiation, and addition or subtraction of a constant to be performed on each value. Instead of the second division with the second integral, for example, division using a specific value of the signal strength of the fourth frequency spectrum may be performed.

For example, in step SP33B, the computation processor 22a corrects the temporary flow calculation value Fp calculated in step SP32B with the D value Vd of the signal strength Pd of the DC component extracted by the extractor 21a in step SP2. In this case, for example, the computation processor 22a divides the temporary flow calculation value Fp calculated in step SP32B with the signal strength Pd of the DC component extracted by the extractor 21a in step SP2 raised to the 2m-th (m is a predetermined positive number) power to calculate the flow calculation value F. This calculation may include, for example, one or more calculations such as multiplication by a coefficient, exponentiation, and addition or subtraction of a constant to be performed on each value.

In this example, if calculating the flow calculation value F based on the fourth frequency spectrum of the AC component included in the signal output from the light receiver 12, the computation processor 22a performs correction using the D value Vd of the strength of the DC component of the signal output from the light receiver 12. Thus, any uniformly reduced strength of the signal output from the light receiver 12 is less likely to change the relationship between the flow calculation value F and the actual flow state of the fluid 2b. Thus, the measurement device 1 can easily have higher measurement accuracy.

1-3. Overview of First Embodiment

The measurement device 1 according to the first embodiment calculates, for example, the flow calculation value F by performing correction using the value (D value) Vd of the signal strength Pd of the DC component and calculation of the frequency spectrum based on the signal output from the light receiver 12. Thus, any uniformly reduced strength of the signal output from the light receiver 12 is less likely to change the relationship between the flow calculation value F and the actual flow state of the fluid 2b. Thus, the measurement device 1 can easily have higher measurement accuracy.

2. Other Embodiments

The present disclosure is not limited to the first embodiment and may be changed or modified in various manners without departing from the spirit and scope of the present disclosure.

2-1. Second Embodiment

As shown in FIG. 10, the measurement device 1 according to the first embodiment may include, for example, an input device 50 or an output device 60.

The input device 50 is connectable to, for example, the controller 20 through the connector 30. In response to, for example, the operation of a user, the input device 50 can input various conditions (also referred to as measurement conditions) on the measurement of the flow quantitative value Vq in the measurement device 1 into the controller 20. Examples of the measurement conditions include the frequency range in the frequency spectrum calculated by the computation processor 22a. Examples of the input device 50 include an operation portion such as a keyboard, a mouse, a touchscreen, and a switch, and a microphone for voice input. The input device 50 allows a user to easily set intended measurement conditions. Thus, the measurement device 1 can enhance user convenience. The measurement conditions may also include, for example, the light quantity or intensity of the illumination light L1 emitted from the light emitter 11, a cycle in which the light receiver 12 outputs a signal, the sampling rate in AD conversion, an operation expression on calibration data and a coefficient in this operation expression, and a coefficient and an exponent in division or subtraction. The input device 50 may also allow input of various sets of information on the fluid 2b such as the viscosity, concentration, or the size of a scatterer in the fluid 2b.

The output device 60 is connectable to, for example, the controller 20 through the connector 30. The output device 60 may include a display that visibly outputs various sets of information on measurements of the flow quantitative value Vq or a speaker that audibly outputs various sets of information on measurements of the flow quantitative value Vq. Examples of the display include a liquid crystal display and a touchscreen. If the input device 50 includes a touchscreen, the displays of the input device 50 and the output device 60 may be a single touchscreen. The measurement device 1 with this structure includes fewer components, is downsized, and facilitates manufacture. A display that can visibly display the measurement conditions, the frequency spectrum, or the flow calculation value F or flow quantitative value Vq as a measurement result allows a user to easily view the various sets of information on measurements of the flow quantitative value Vq. For example, the display may allow a user to change the output form of various sets of information in the output device 60 through the input device 50. The change in the output form may include, for example, a change in the display form and switching of displayed information. The display thus allows a user to easily view the various sets of information on measurements of the flow quantitative value Vq. Thus, the measurement device 1 can enhance user convenience.

2-2. Third Embodiment

As shown in FIG. 11, the measurement device 1 according to each embodiment may also include, for example, an external controller 70. The external controller 70 may include, for example, a computer such as a microcomputer.
The external controller 70 holds measurement conditions such as the light quantity or intensity of the illumination light L1, a cycle in which the light receiver 12 outputs a signal, and the sampling rate in AD conversion. These measurement conditions may be input into the controller 20. Thus, the processes to be performed by the computation processor 22a can be reduced, and the controller 20 can improve the processing speed. The measurement conditions include, for example, the same conditions as the various conditions on the measurement of the flow quantitative value Vq in the measurement device 1. The various conditions can be input by the input device 50.
The external controller 70 may control, for example, the input device 50 and the output device 60. This structure reduces units having various functions (also referred to as functional units) controlled by the controller 20, and thus can improve the processing speed of the controller 20. The external controller 70 may include, for example, various other functional units including multiple electronic components. Examples of the various other functional units include a pressure gauge and a thermometer. Thus, the measurement device 1 can, for example, enhance design flexibility and user convenience.
The external controller 70, the controller 20, the input device 50, and the output device 60 may communicate with one another with wires or wirelessly. The controller 20 and the external controller 70 communicate with each other in accordance with, for example, any telecommunications standard. Such telecommunications standards include Inter-Integrated Circuit (IIC), the Serial Peripheral Interface (SPI), and a universal asynchronous receiver-transmitter (UART).
The sensor 10, the signal processor 21, and the external controller 70 may directly communicate with one another. In this case, the measurement device 1 may eliminate the controller 20, and the external controller 70 may serve as the controller 20. For example, the sensor 10 and the external controller 70 may communicate directly with each other to eliminate delays of signals between the controller 20 and the external controller 70. The measurement device 1 can thus improve the processing speed and enhance user convenience.

2-3. Fourth Embodiment

In each of the above embodiments, a measurement system 200 may include all the components or at least two components of the measurement device 1 connected to allow communication between them. As shown in FIG. 12, for example, the measurement system 200 according to the fourth embodiment includes the light emitter 11, the light receiver 12, the signal processor 21 including the extractor 21a, and the information processor 22 including the computation processor 22a. In the example shown in FIG. 12, the light emitter 11 and the light receiver 12, the light emitter 11 and the information processor 22, the light receiver 12 and the signal processor 21, and the signal processor 21 and the information processor 22 are connected to allow communication between them.

2-4. Fifth Embodiment

In each of the embodiments, the predetermined exponent m in Formula 3 may be changed as appropriate in accordance with factors of uniformly reducing the strength of the signal output from the light receiver 12 (strength reduction factors). Examples of strength reduction factors include the intensity of the illumination light L1 described above, the thickness, the inner diameter, and the material of the flow passage component 2a defining the flow passage of the fluid 2b, the particle concentration and light absorptivity in the fluid 2b, and the positional or orientational relationship between the light emitter 11, the flow passage component 2a, and the light receiver 12.
In this example, the measurement device 1 sets the particle concentration in the fluid 2b to a first concentration of 10, a second concentration of 7 or 70% of the first concentration, and a third concentration of 3 or 30% of the first concentration, using the quantitative value (flow quantitative value) Vq on the flow state of the fluid 2b flowing through a transparent tube serving as the flow passage component 2a set to a predetermined value with, for example, a pump. In this example, for the fluid 2b with the particle concentration of the first concentration, the measurement device 1 can obtain the first frequency spectrum P1(f) indicated with a curve Ln61 drawn with a bold solid line curve in FIG. 13A. For the fluid 2b with the particle concentration of the second concentration, for example, the measurement device 1 can obtain the first frequency spectrum P1(f) indicated with a curve Ln62 drawn with a bold dot-dash line in FIG. 13A. For the fluid 2b with the particle concentration of the third concentration, for example, the measurement device 1 can obtain the first frequency spectrum P1(f) indicated with a curve Ln63 drawn with a bold two-dot chain line in FIG. 13A. As shown in FIG. 13A, the strength of the first frequency spectrum P1(f) is reduced with the reduced particle concentration in the fluid 2b.

In contrast, for example, the measurement device 1 divides the first frequency spectrum P1(f) by the D value Vd of the signal strength Pd of the DC component to calculate the corrected frequency spectrum (second frequency spectrum) P2(f). In this case, as shown in FIG. 13B, the second frequency spectra P2(f) are almost the same independently of the particle concentration in the fluid 2b unlike the first frequency spectra P1(f) shown in FIG. 13A. For example, a curve Ln71 drawn with a bold solid line shown in FIG. 13B indicates a second frequency spectrum P2(f) for the fluid 2b with the particle concentration of the first concentration. For example, a curve Ln72 drawn with a bold dot-dash line in FIG. 13B indicates a second frequency spectrum P2(f) for the fluid 2b with the particle concentration of the second concentration. For example, a curve Ln73 drawn with a bold two-dot chain line in FIG. 13B indicates a second frequency spectrum P2(f) for the fluid 2b with the particle concentration of the third concentration.

In this case, for example, the particle concentration in the fluid 2b serves as the strength reduction factor and the predetermined exponent m is set to 2.

The predetermined exponent m may be determined based on, for example, the experimental measurements obtained by the measurement device 1 at specific timing or by simulation. Examples of specific timing include time before shipment of the measurement device 1 or time at the maintenance of the measurement device 1. The predetermined exponent m may be determined based on the experimental measurements with the method described below. For example, the quantitative value (flow quantitative value) Vq on the flow state of the fluid 2b flowing through a transparent tube serving as the flow passage component 2a is set to a predetermined value with, for example, a pump. The numerical value of a specific strength reduction factor causing uniformly reduced strength of the signal output from the light receiver 12 is sequentially set to the multiple reference values. The measurement device 1 then performs measurements. In this state, for example, the first frequency spectrum P1(f) for the AC component of the signal output from the light receiver 12 is calculated for each of the reference values, and the strength Pd of the DC component of the signal output from the light receiver 12 is obtained for each of the reference values. The predetermined exponent m is determined based on the combination of the strength Pd of the DC component and the first frequency spectrum P1(f) obtained for each of the reference values.

2-5. Sixth Embodiment

In each embodiment, for example, the computation processor 22a may calculate a frequency spectrum P(f) for the signal output from the light receiver 12 at the temporal change in the signal strength, and calculate the flow quantitative value Vq with a computation using a value based on the frequency spectrum P(f) and the value (D value) Vd of the signal strength Pd of the DC component. In such a structure as well, the measurement device 1 can have higher measurement accuracy.

The value based on the frequency spectrum P(f) may be, for example, the flow calculation value F calculated in each embodiment, or the flow calculation value F serving as a value of the strength based on the frequency spectrum P(f). In this case, for example, the flow calculation value F may be, for the frequency spectrum P(f), an integral in a predetermined frequency range, a specific frequency component, specific strength, or a combination of two or more of these values. For example, an integral ($\int$P(f)df) calculated for the frequency spectrum P(f) is used as the integral in the predetermined frequency range. For example, the strength of a predetermined frequency in the frequency spectrum P(f) is used as the specific frequency component. For example, a fixed frequency or an intermediate frequency of the frequency spectrum P(f) is used as the specific frequency. For example, a boundary frequency at which an integral of the strength of the lower frequencies and an integral of the strength of the higher frequencies in the frequency spectrum P(f) have a predetermined ratio is used as the intermediate frequency. For example, the predetermined ratio is set to 1:1. For example, a maximum value of the intensity in the frequency spectrum P(f) is used as the specific intensity. Examples of the combination of two or more values include a sum of the integral and the specific frequency component, and a sum of or difference between the specific frequency component and the specific intensity.

The computation processor 22a can calculate a flow quantitative value Vq based on the flow calculation value F, the value (D value) Vd of the signal strength Pd of the DC component, and calibration data (a calibration curve) prepared in advance. If, for example, the calibration data on the flow rate of the fluid 2b is prepared in advance, the flow rate of the fluid 2b can be calculated based on the flow calculation value F, the D value Vd, and the calibration curve of the flow rate serving as the flow quantitative value Vq. If, for example, the calibration data on the flow velocity of the fluid 2b is prepared in advance, the flow velocity of the fluid 2b can be calculated based on the flow calculation value F, the D value Vd, and the calibration curve of the flow velocity serving as the flow quantitative value Vq. Thus, at least one of the flow rate or the flow velocity of the fluid 2b can be calculated. As described above, for example, any uniformly reduced strength of the signal output from the light receiver 12 is less likely to change the relationship between the flow calculation value F and the actual flow state of the fluid 2b. Thus, the measurement device 1 can have higher measurement accuracy.

For example, the calibration data may be stored in the storage 22b in advance before the flow quantitative value Vq of the fluid 2b is measured. The calibration data may be stored in the form of, for example, a functional formula or a table.

The calibration data can be prepared by, for example, the measurement device 1 calculating the flow calculation value F of the fluid 2b, as a measurement target, flowing through the flow passage component 2a at a known flow quantitative value Vq while switching strength reduction factors from one another. The calculation of the flow calculation value F performed by the measurement device 1 includes the light emitter 11 illuminating the illumination target 2 with the illumination light L1, the light receiver 12 receiving the coherent light L2 including light scattered by the illumination target 2, and the computation processor 22a calculating the flow calculation value F. For example, the measurement device 1 calculates the flow calculation value F of the fluid 2b flowing through the flow passage component 2a at a known flow quantitative value Vq, and derives calibration data based on the relationship between the known flow quantitative value Vq, the flow calculation value F, and the D value Vd. More specifically, for example, an operation expression (calibration curve) including the flow calculation value F as a parameter and a coefficient that changes with the D value Vd is derived as calibration data.

For example, the calibration curve is written by Formula 10 including the flow quantitative value Vq denoted with y, the flow calculation value F denoted with x, coefficients a(z) and b(z) that change with z serving as the D value Vd, and a variable c(z). The D value Vd may be, for example, the same as the signal strength Pd of the DC component, or may be obtained by calculation such as multiplication of the signal strength Pd of the DC component by a coefficient.

$$y = a(z) \times x^2 + b(z) \times x + c(z) \qquad (10)$$

The coefficient a(z) is, for example, defined with Formula 11 including coefficients a1 and b1 and a constant c1. The coefficient b(z) is, for example, defined with Formula 12 including coefficients a2 and b2 and a constant c2. The variable c(z) is, for example, defined with Formula 13 including coefficients a3 and b3 and a constant c3.

$$a(z) = a1 \times z^2 + b1 \times z + c1 \qquad (11)$$

$$b(z) = a2 \times z^2 + b2 \times z + c2 \qquad (12)$$

$$c(z) = a3 \times z^2 + b3 \times z + c3 \qquad (13)$$

The six coefficients a1, b1, a2, b2, a3, and b3 and the three constants c1, c2, and c3 can be set, for example, in the manner described below.

For example, the D value Vd of the signal strength Pd of the DC component is defined as a first D value Vd1 by setting the strength reduction factor in a first state. The flow calculation value F of the fluid 2b flowing through the flow passage component 2a at the flow quantitative value Vq of a known value y1 is calculated as a value x1, the flow calculation value F of the fluid 2b flowing through the flow passage component 2a at the flow quantitative value Vq of a known value y2 is calculated as a value x2, and the flow calculation value F of the fluid 2b flowing through the flow passage component 2a at the flow quantitative value Vq of a known value y3 is calculated as a value x3. In this case, Formulas 14 to 16 below are obtained.

$$y1 = a(Vd1) \times x1^2 + b(Vd1) \times x1 + c(Vd1) \qquad (14)$$

$$y2 = a(Vd1) \times x2^2 + b(Vd1) \times x2 + c(Vd1) \qquad (15)$$

$$y3 = a(Vd1) \times x3^2 + b(Vd1) \times x3 + c(Vd1) \qquad (16)$$

Based on Formulas 14, 15, and 16, a coefficient a(Vd1), a coefficient b(Vd1), and a variable c(Vd1) for the D value Vd of the first D value Vd1 are calculated. Thus, Formulas 17 to 19 below are obtained.

$$a(Vd1) = a1 \times Vd1^2 + b1 \times Vd1 + c1 \qquad (17)$$

$$b(Vd1) = a2 \times Vd1^2 + b2 \times Vd1 + c2 \qquad (18)$$

$$c(Vd1) = a3 \times Vd1^2 + b3 \times Vd1 + c3 \qquad (19)$$

For example, the D value Vd is defined as a second D value Vd2 by setting the strength reduction factor in a second state. The flow calculation value F of the fluid 2b flowing through the flow passage component 2a at the flow quantitative value Vq of a known value y4 is calculated as a value x4, the flow calculation value F of the fluid 2b flowing through the flow passage component 2a at the flow quantitative value Vq of a known value y5 is calculated as a value x5, and the flow calculation value F of the fluid 2b flowing through the flow passage component 2a at the flow quantitative value Vq of a known value y6 is calculated as a value x6. In this case, Formulas 20 to 22 below are obtained.

$$y4 = a(Vd2) \times x4^2 + b(Vd2) \times x4 + c(Vd2) \qquad (20)$$

$$y5 = a(Vd2) \times x5^2 + b(Vd2) \times x5 + c(Vd2) \qquad (21)$$

$$y6 = a(Vd2) \times x6^2 + b(Vd2) \times x6 + c(Vd2) \qquad (22)$$

Based on Formulas 20, 21, and 22, a coefficient a(Vd2), a coefficient b(Vd2), and a variable c(Vd2) for the D value Vd of the second D value Vd2 are calculated. Thus, Formulas 23 to 25 below are obtained.

$$a(Vd2) = a1 \times Vd2^2 + b1 \times Vd2 + c1 \qquad (23)$$

$$b(Vd2) = a2 \times Vd2^2 + b2 \times Vd2 + c2 \qquad (24)$$

$$c(Vd2) = a3 \times Vd2^2 + b3 \times Vd2 + c3 \qquad (25)$$

For example, the D value Vd is defined as a second D value Vd3 by setting the strength reduction factor in a third state. The flow calculation value F of the fluid 2b flowing through the flow passage component 2a at the flow quantitative value Vq of a known value y7 is calculated as a value x7, the flow calculation value F of the fluid 2b flowing through the flow passage component 2a at the flow quantitative value Vq of a known value y8 is calculated as a value x8, and the flow calculation value F of the fluid 2b flowing through the flow passage component 2a at the flow quantitative value Vq of a known value y9 is calculated as a value x9. In this case, Formulas 26 to 28 below are obtained.

$$y7 = a(Vd3) \times x7^2 + b(Vd3) \times x7 + c(Vd3) \qquad (26)$$

$$y8 = a(Vd3) \times x8^2 + b(Vd3) \times x8 + c(Vd3) \qquad (27)$$

$$y9 = a(Vd3) \times x9^2 + b(Vd3) \times x9 + c(Vd3) \qquad (28)$$

Based on Formulas 26, 27, and 28, a coefficient $a(Vd3)$, a coefficient $b(Vd3)$, and a variable $c(Vd3)$ for the D value Vd of the third D value Vd3 are calculated. Thus, Formulas 29 to 31 below are obtained.

$$a(Vd3) = a1 \times Vd3^2 + b1 \times Vd3 + c1 \qquad (29)$$

$$b(Vd3) = a2 \times Vd3^2 + b2 \times Vd3 + c2 \qquad (30)$$

$$c(Vd3) = a3 \times Vd3^2 + b3 \times Vd3 + c3 \qquad (31)$$

Based on Formulas 17 to 19, Formulas 23 to 25, and Formulas 29 to 31, the six coefficients a1, b1, a2, b2, a3, and b3 and the three constants c1, c2, and c3 are calculated. The calculated six coefficients a1, b1, a2, b2, a3, and b3 and the calculated three constants c1, c2, and c3 are substituted into three Formulas 11 to 13, and thus the calibration data indicating operation expressions (calibration curves) defined by Formulas 10 to 13 can be obtained.

The functional formula representing the calibration curve may be, for example, written using a polynomial expression including an m-th order term (m is a natural number greater than or equal to 2), where the flow quantitative value Vq is denoted with y and the flow calculation value F is a variable x. The functional formula defining the coefficients and the variables in the functional formula representing the calibration curve may be, for example, written using a polynomial expression including an n-th order term (n is a natural number greater than or equal to 2), where the D value Vd is a variable z. The functional formula representing the calibration curve may include, for example, at least one term selected from the term of logarithm and the term of exponentiation of a variable x serving as the flow calculation value F, or include a coefficient unchangeable by the D value Vd. The functional formula defining the coefficients in the functional formula representing the calibration curve may include, for example, at least one term selected from the term of logarithm and the term of exponentiation of a variable z serving as the D value Vd, or include a coefficient unchangeable by the D value Vd. In other words, for example, the functional formula may calculate the flow quantitative value Vq with a computation based on the flow calculation value F and a coefficient that changes with the D value Vd. In still other words, for example, the computation processor 22a may calculate the flow quantitative value Vq based on the flow calculation value F and a coefficient corresponding to the value (D value) Vd of the signal strength Pd of the DC component.

3. Others

In each embodiment, the computation processor 22a may calculate, for example, the frequency spectrum P(f) for a signal including the AC and DC components after the signal processor 21 processes the signal output from the light receiver 12. In this case as well, the computation processor 22a can calculate the frequency spectrum P(f) for the AC component of the signal output from the light receiver 12.

In the first to fifth embodiments, for example, a value corresponding to the mean frequency fm is used for calculating the flow calculation value F, but the value is not limited to this example. For example, instead of the value corresponding to the mean frequency fm, a specific value of a frequency for the frequency spectrum P(f) may be used. A boundary frequency at which an integral of the strength of lower frequencies and an integral of the strength of higher frequencies in the frequency spectrum P(f) having a predetermined ratio may be used as an example specific value of the frequency. For example, the predetermined ratio is set to 1:1. A frequency with any strength within a frequency range including the frequency with a maximum strength value for the frequency spectrum P(f) may be used as an example specific value of the frequency. A frequency with a maximum strength value for the frequency spectrum P(f) may be used as an example specific value of the frequency. A frequency of any inclination in a frequency range including a frequency having an absolute value of inclination of a strength change with a minimum value for the frequency spectrum P(f) may be used as an example specific value of the frequency. A frequency having an absolute value of inclination of a strength change with a minimum value for the frequency spectrum P(f) may be used as an example specific value of the frequency.

In the first to fifth embodiments, the computation processor 22a may eliminate a calculation of the flow quantitative value Vq based on the flow calculation value F. The structure also enables a user to monitor a change in the flow state of the fluid 2b based on the change in the flow calculation value F. Thus, the measurement device 1 can have higher measurement accuracy.

In each embodiment, at least one of the functions of the computation processor 22a may be implemented in hardware such as a dedicated electronic circuit.

The components described in the above embodiments and modifications may be entirely or partially combined as appropriate unless any contradiction arises.

Reference Signs List

| | |
|---|---|
| 1 | measurement device |
| 2 | illumination target |
| 2a | flow passage component |
| 2b | fluid |
| 2i | internal space |
| 10 | sensor |
| 11 | light emitter |
| 12 | light receiver |
| 20 | controller |
| 21 | signal processor |
| 21a | extractor |
| 21b | amplifier |
| 22 | information processor |
| 22a | computation processor |
| 22b | storage |
| 30 | connector |
| 50 | input device |
| 60 | output device |
| 70 | external controller |
| 200 | measurement system |
| L1 | illumination light |
| L2 | coherent light |
| PG1 | program |

**Claims**

1. A measurement device, comprising:

   a light emitter configured to illuminate an illumination target having an internal space through which a fluid flows;
   a light receiver configured to receive coherent light including light scattered by the illumination target and to output a signal corresponding to intensity of the coherent light;
   an extractor configured to extract a direct-current component from the signal output from the light receiver at a temporal change in strength of the signal; and
   a processor configured to calculate a calculation value for a flow state of the fluid by performing a process on the signal output from the light receiver, the process including correction using a value of signal strength of the direct-current component and calculation of a frequency spectrum for the signal at the temporal change in the signal strength.

2. The measurement device according to claim 1, wherein
   the correction includes division using the value of the signal strength of the direct-current component.

3. The measurement device according to claim 1 or claim 2, wherein
   the processor calculates a first frequency spectrum for the signal output from the light receiver at the temporal change in the signal strength, calculates a corrected second frequency spectrum with correction of signal strength in the first frequency spectrum using the value of the signal strength of the direct-current component, and calculates the calculation value based on the corrected second frequency spectrum.

4. The measurement device according to claim 1 or claim 2, wherein
the processor at least corrects signal strength of an alternating current component included in the signal output from the light receiver using the value of the signal strength of the direct-current component, calculates a third frequency spectrum for signal strength of a corrected alternating current component, and calculates the calculation value based on the third frequency spectrum.

5. The measurement device according to claim 1 or claim 2, wherein
the processor calculates a fourth frequency spectrum for the signal output from the light receiver at the temporal change in the signal strength, and calculates the calculation value with a computation including correction using the value of the signal strength of the direct-current component based on the fourth frequency spectrum.

6. The measurement device according to any one of claims 1 to 5, wherein
the processor calculates a quantitative value for the flow state of the fluid based on the calculation value.

7. The measurement device according to claim 6, wherein
the processor calculates the quantitative value based on the calculation value and a coefficient corresponding to the value of the signal strength of the direct-current component.

8. A measurement device, comprising:

a light emitter configured to illuminate an illumination target having an internal space through which a fluid flows;
a light receiver configured to receive coherent light including light scattered by the illumination target and to output a signal corresponding to intensity of the coherent light;
an extractor configured to extract a direct-current component from the signal output from the light receiver at a temporal change in strength of the signal; and
a processor configured to calculate a frequency spectrum for the signal output from the light receiver at the temporal change in the signal strength and to calculate a quantitative value for a flow state of the fluid with a computation using a value of signal strength based on the frequency spectrum and a value of signal strength of the direct-current component.

9. A measurement system, comprising:

a light emitter configured to illuminate an illumination target having an internal space through which a fluid flows;
a light receiver configured to receive coherent light including light scattered by the illumination target and to output a signal corresponding to intensity of the coherent light;
an extractor configured to extract a direct-current component from the signal output from the light receiver at a temporal change in strength of the signal; and
a processor configured to calculate a calculation value for a flow state of the fluid by performing a process on the signal output from the light receiver, the process including correction using a value of signal strength of the direct-current component and calculation of a frequency spectrum for the signal at the temporal change in the signal strength.

10. A measurement system, comprising:

a light emitter configured to illuminate an illumination target having an internal space through which a fluid flows;
a light receiver configured to receive coherent light including light scattered by the illumination target and to output a signal corresponding to intensity of the coherent light;
an extractor configured to extract a direct-current component from the signal output from the light receiver at a temporal change in strength of the signal; and
a processor configured to calculate a frequency spectrum for the signal output from the light receiver at the temporal change in the signal strength and to calculate a quantitative value for a flow state of the fluid with a computation using a value of signal strength based on the frequency spectrum and a value of signal strength of the direct-current component.

11. A measurement method, comprising:

illuminating, with a light emitter, an illumination target having an internal space through which a fluid flows, receiving, with a light receiver, coherent light including light scattered by the illumination target, and outputting, with the light receiver, a signal corresponding to intensity of the coherent light;

extracting, with an extractor, a direct-current component from the signal output from the light receiver at a temporal change in strength of the signal; and

calculating, with a processor, a calculation value for a flow state of the fluid by performing a process on the signal output from the light receiver, the process including correction using a value of signal strength of the direct-current component extracted by the extractor and calculation of a frequency spectrum for the signal at the temporal change in the signal strength.

12. A measurement method, comprising:

illuminating, with a light emitter, an illumination target having an internal space through which a fluid flows, receiving, with a light receiver, coherent light including light scattered by the illumination target, and outputting, with the light receiver, a signal corresponding to intensity of the coherent light;

extracting, with an extractor, a direct-current component from the signal output from the light receiver at a temporal change in strength of the signal; and

calculating, with a processor, a frequency spectrum for the signal output from the light receiver at the temporal change in the signal strength, and calculating, with the processor, a quantitative value for a flow state of the fluid with a computation using a value of signal strength based on the frequency spectrum and a value of signal strength of the direct-current component extracted by the extractor.

13. A program executable by a processor included in a measurement device to cause the measurement device to function as the measurement device according to any one of claims 1 to 8.

# FIG. 1

FIG. 2

## FIG. 3A

## FIG. 3B

FIG. 4A

Ln11

Ln12

Ln13

Signal strength

Frequency

FIG. 4B

Ln14

Signal strength of direct-current component

Time

FIG. 5A

FIG. 5B

FIG. 6A

FIG. 6B

FIG. 7A

Start

Output signal in accordance with illumination ⎯ SP1

Process signal (including extraction of direct-current component) ⎯ SP2

Calculate flow calculation value ⎯ SP3

Calculate flow quantitative value ⎯ SP4

End

FIG. 7B

Start

Calculate first frequency spectrum ⎯ SP31

Correct using signal strength of direct-current component (calculate second frequency spectrum) ⎯ SP32

Calculate flow calculation value ⎯ SP33

End

FIG. 8

```
                    ┌─────────────┐
                    │    Start    │
                    └──────┬──────┘
                           │
            ┌──────────────▼──────────────────────────┐
            │ Correct signal output from light receiver using │ ──── SP31A
            │  signal strength of direct-current component     │
            └──────────────┬──────────────────────────┘
                           │
            ┌──────────────▼──────────────────────────┐
            │       Calculate frequency spectrum       │ ──── SP32A
            └──────────────┬──────────────────────────┘
                           │
            ┌──────────────▼──────────────────────────┐
            │       Calculate flow calculation value    │ ──── SP33A
            └──────────────┬──────────────────────────┘
                           │
                    ┌──────▼──────┐
                    │     End     │
                    └─────────────┘
```

FIG. 9

```
                    ┌─────────────┐
                    │    Start    │
                    └──────┬──────┘
                           │
            ┌──────────────▼──────────────────────────┐
            │       Calculate frequency spectrum       │ ──── SP31B
            └──────────────┬──────────────────────────┘
                           │
            ┌──────────────▼──────────────────────────┐
            │  Calculate temporary flow calculation value │ ──── SP32B
            └──────────────┬──────────────────────────┘
                           │
            ┌──────────────▼──────────────────────────┐
            │  Calculate flow calculation value (correct with │ ──── SP33B
            │   signal strength of direct-current component)  │
            └──────────────┬──────────────────────────┘
                           │
                    ┌──────▼──────┐
                    │     End     │
                    └─────────────┘
```

FIG. 10

# FIG. 11

# FIG. 12

200

**11** Light emitter

**12** Light receiver

Signal processor — 21

Extractor — 21a

Amplifier — 21b

— 22

Computation processor — 22a

Storage

PG1 — Program — 22b

FIG. 13A

FIG. 13B

FIG. 14A

FIG. 14B

FIG. 15

FIG. 16A

FIG. 16B

**INTERNATIONAL SEARCH REPORT**

| International application No. |
| --- |
| PCT/JP2020/040593 |

**A. CLASSIFICATION OF SUBJECT MATTER**
G01F 1/66(2006.01)i; A61B 5/026(2006.01)i; A61B 5/0285(2006.01)i
FI: G01F1/66 103; A61B5/026 120; A61B5/0285 H

According to International Patent Classification (IPC) or to both national classification and IPC

**B. FIELDS SEARCHED**

Minimum documentation searched (classification system followed by classification symbols)
G01F1/00-9/02; G01P5/00-5/26; A61B5/02-5/03

Documentation searched other than minimum documentation to the extent that such documents are included in the fields searched
Published examined utility model applications of Japan        1922–1996
Published unexamined utility model applications of Japan      1971–2021
Registered utility model specifications of Japan              1996–2021
Published registered utility model applications of Japan      1994–2021

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| X | JP 2008-278993 A (NIPPON TELEGRAPH AND TELEPHONE CORP.) 20 November 2008 (2008-11-20) paragraphs [0017]-[0040], fig. 1-9 | 1-4, 6-7, 9, 11, 13 |
| X | WO 2019/146762 A1 (KYOCERA CORP.) 01 August 2019 (2019-08-01) paragraphs [0009]-[0057], fig. 1-6 | 1-2, 5-7, 9, 11, 13 |
| X | WO 2013/153664 A1 (PIONEER CORP.) 17 October 2013 (2013-10-17) paragraphs [0016], [0049]-[0114], fig. 1-7 | 8, 10, 12-13 |
| X | JP 2017-187359 A (NIPPON TELEGRAPH AND TELEPHONE CORP.) 12 October 2017 (2017-10-12) paragraphs [0022]-[0063], fig. 1-11 | 8, 10, 12-13 |

☐ Further documents are listed in the continuation of Box C.      ☒ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 07 January 2021 (07.01.2021) | 26 January 2021 (26.01.2021) |

| Name and mailing address of the ISA/ | Authorized officer |
| --- | --- |
| Japan Patent Office | |
| 3-4-3, Kasumigaseki, Chiyoda-ku, | |
| Tokyo 100-8915, Japan | Telephone No. |

Form PCT/ISA/210 (second sheet) (January 2015)

**INTERNATIONAL SEARCH REPORT**
Information on patent family members

International application No.

PCT/JP2020/040593

| Patent Documents referred in the Report | Publication Date | Patent Family | Publication Date |
|---|---|---|---|
| JP 2008-278993 A | 20 Nov. 2008 | (Family: none) | |
| WO 2019/146762 A1 | 01 Aug. 2019 | JP 6746002 B2 | |
| WO 2013/153664 A1 | 17 Oct. 2013 | JP 5806390 B2<br>EP 2837327 A1<br>paragraphs [0016],<br>[0050]-[0111], fig.<br>1-7 | |
| JP 2017-187359 A | 12 Oct. 2017 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 5806390 B